(19)

**Europäisches Patentamt**
**European Patent Office**
**Office européen des brevets**

(11)  **EP 4 623 692 A1**

(12)  # EUROPEAN PATENT APPLICATION

(43) Date of publication:
**01.10.2025 Bulletin 2025/40**

(21) Application number: **24167956.2**

(22) Date of filing: **31.03.2024**

(51) International Patent Classification (IPC):
**A01N 63/40** (2020.01)     **A01P 1/00** (2006.01)
**C12N 7/00** (2006.01)

(52) Cooperative Patent Classification (CPC):
(C-Sets available)
**A01N 63/40; A01P 1/00; C12N 7/00;**
C12N 2795/10021; C12N 2795/10032     (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(83) **Declaration under Rule 32(1) EPC (expert
solution)**

(71) Applicants:
• **Enviroinvest Környezetvédelmi és
Biotechnológiai
Zártkörüen Müködö Részvénytárasaság
7632 Pécs (HU)**
• **Asociacion para la Promocion de la Pera de
Rincon de Soto
26550 Rincon de Soto (ES)**
• **Kimitec Biogroup S.L
04738 Vícar Almeria (ES)**

(72) Inventors:
• **GOMES GERVÁSIO, Kellen Katiúsce
04738 Vicar, Almeria (ES)**

• **TORRES CORTÉS, Gloria
04738 Vicar, Almeria (ES)**
• **FIESELER, Lars
8820 Wädenswil (CH)**
• **KAMMERECKER, Sandrine
8820 Wädenswil (CH)**
• **GAYDAR, Steven
8820 Wädenswil (CH)**
• **LOMADZE, Elene
7633 Pécs (HU)**
• **KÖRÖSINÉ PAPP, Szilvia
7668 Keszü (HU)**
• **KOVÁCS, Tamás
7761 Kozármisleny (HU)**

(74) Representative: **Danubia Patent & Law Office LLC
Bajcsy-Zsilinszky út 16
1051 Budapest (HU)**

Remarks:
•The complete document including Reference
Table(s) and the Sequence Listing(s) can be
downloaded from the EPO website
•Claims 16 to 18, 20 to 22 and 24 to 26 are deemed
to be abandoned due to non-payment of the claims
fees (Rule 45(3) EPC).

(54)  **BACTERIOPHAGES AGAINST ERWINIA AMYLOVORA FIRE BLIGHT**

(57)     The invention relates to isolated bacteriophages and a mixtures thereof, capable of infecting and lysing *Erwinia amylovora*. The invention also relates to compositions comprising the bacteriophages and their mixtures (cocktails), process for the production of the bacteriophage and uses of the compositions. Furthermore, the invention relates to a method for the prevention or treatment of infection of plants by the bacterium *Erwinia amylovora*.

Figure 10.d

**EP 4 623 692 A1**

(52) Cooperative Patent Classification (CPC): (Cont.)

C-Sets
**A01N 63/40, A01N 63/40**

**Description**

FIELD OF THE INVENTION

The invention relates to isolated bacteriophages and a mixtures thereof, capable of infecting *Erwinia amylovora.* The invention also relates to compositions comprising the bacteriophages and their mixtures (cocktails), process for the production of the bacteriophage and uses of the compositions. Furthermore, a method for the prevention or treatment of infection of plants by the bacterium *Erwinia amylovora.*

BACKGROUND ART

*Erwinia amylovora,* the causative agent of fire blight, is one such devastating bacterium that affects a wide range of fruit trees and ornamental plants such as apples, pears, quince, and other fruits from the Rosaceae family. These plant diseases caused by bacterial pathogens have emerged as significant challenges in agriculture, threatening global food security and economic stability [Momol, M. T. and Aldwinckle, H. S. (2000) 'Genetic diversity and host range of Erwinia amylovora.', CABI Books. CABI.]. In Hungary, *Erwinia amylovora* was first detected in apple orchards in 1996 [Hevesi, M. (1996). Az Erwinia amylovora (Burill) Winslow et al. hazai megjelenése almán. Növényvédelem, 32(5), 225-228], since *Erwinia amylovora* had been detected from different plants and various areas[Végh, A., Hevesi, M., Pájtli, E. et al. Characterization of Erwinia amylovora strains from Hungary. Eur J Plant Pathol 147, 455-461 (2017).].

*Erwinia amylovora*'s infection symptoms include wilting, cankers, necrosis, and oozing bacterial exudates, leading to extensive losses in fruit production and tree health [Vanneste, J.L., and Eden-Green, S. (2000) Migration of Erwinia amylovora in host plant tissues. In Fire Blight: The Disease and its Causative Agent, Erwinia amylovora, Vanneste, J.L. (ed). Wallingford: CAB International, pp. 73-83.; Skoneczny, H.; Kubiak, K.; Spiralski, M.; Kotlarz, J.; Mikiciński, A.; Pulawska, J. Fire Blight Disease Detection for Apple Trees: Hyperspectral Analysis of Healthy, Infected and Dry Leaves. Remote Sens. 2020, 12, 2101.]. Conventional control methods involving chemical sprays and cultural practices have limitations, such as the development of resistance in bacteria and their detrimental effects on beneficial microorganisms [Zum, J.D.; Norelli, J.L.; Montanari, S.; Bell, R.; Bassil, N.V. Dissecting Genetic Resistance to Fire Blight in Three Pear Populations.Phytopathology 2020, 110, 1305-1311.; Escursell, M.M.; Roschi, A.; Smits, T.H.M.; Rezzonico, F. Characterization and Direct Molecular Discrimination of RpsL Mutations Leading to High Streptomycin Resistance in Erwinia Amylovora. J Plant Pathol 2021, 103, 99-108.] The search for alternative approaches has led researchers to investigate the potential of bacteriophages as natural enemies of *Erwinia amylovora.*

Bacteriophages, the viruses that infect and replicate within bacteria, have garnered considerable interest as potential biocontrol agents against bacterial pathogens [Seed, K. D. (2015). Battling phages: how bacteria defend against viral attack. PLoS Pathog. 11:e1004847.]. These naturally occurring predators have evolved alongside bacteria and possess the ability to specifically target and kill pathogenic strains while leaving beneficial microorganisms and the environment unharmed [Goodridge, L.D.; Abedon, S.T. Bacteriophage Biocontrol: The Technology Matures. Microbiol. Aust. 2008, 29, 48-49.; Polaska, M.; Sokolowska, B. Bacteriophages-A New Hope or a Huge Problem in the Food Industry. AIMS Microbiol. 2019, 5, 324]. The unique host-specificity and self-replicating properties make bacteriophages attractive candidates for the development of targeted and sustainable approaches against *Erwinia amylovora* infections.

Bacteriophages can infect bacteria by attaching to specific receptor sites on their surface and injecting their genetic material into the host cell. Subsequently, they exploit the bacterial machinery, diverting it towards the production of new viral progeny, eventually leading to the lysis and release of multiple phages. This lytic cycle holds immense potential for the targeted eradication of pathogenic bacteria, offering a sustainable and eco-friendly alternative to conventional control methods.

In recent years, several studies have focused on the isolation, characterization, and application of bacteriophages for the control of *Erwinia amylovora.* These phages exhibit high specificity towards the pathogen, efficiently reducing bacterial populations in laboratory and field conditions [Sieiro, C. Areal-Hermida, L. et al. A hundred years of bacteriophages: Can phages replace antibiotics in agriculture and aquaculture? Antibiotics 2020, 9, 493. Svircev, A.; Roach, D.; Castle, A. Framing the future with bacteriophages in agriculture. Viruses 2018, 10, 218.; Jamal, M.; Bukhari, S.M.A.U.S. et al., Bacteriophages: An overview of the control strategies against multiple bacterial infections in different fields. J. Basic Microbiol. 2019, 59, 123-133.]. In Hungary as well were isolated bacteriophages against *Erwinia Amylovora* [Dömötör, D., Becságh, P., Rákhely, G., Schneider, G., & Kovács,T. (2012). Complete genomic sequence of Erwinia amylovora phage PhiEaH2. Journal of Virology, 86(19), 10899-10899.; Meczker, K., Dömötör, D., Vass, J., Rákhely, G., Schneider, G., & Kovács, T. (2014). The genome of the Erwinia amylovora phage PhiEaH1 reveals greater diversity and broadens the applicability of phages for the treatment of fire blight. FEMS Microbiology Letters, 350(1), 25-27. 22.; Schwarczinger, I; Kolozsváriné Nagy, J. et al. (2017) Characterization of Myoviridae and Podoviridae family bacteriophages of Erwinia amylovora from Hungary-potential of application in biological control of fire blight. European Journal of Plant Pathology, 149 (3). pp. 639-652.]. Bacteriophage cocktails containing several phages have been developed to counteract the emergence of bacterial resistance [Kim, H.J.; Jun, J.W. et al. Bacteriophage cocktail for the prevention of multiple-antibiotic-resistant and mono-phage-resistant Vibrio coralliilyticus infection in pacific oyster (Crassostrea gigas) larvae. Pathogens 2020, 9, 831.; Kim, S.G. and Lee, S.B. et al. Phage cocktail in combination with kasugamycin as a potential treatment for fire blight caused by Erwinia amylovora. Antibiotics 2022, 11, 1566.]. The cocktail formulations provide a broader spectrum of activity, targeting different strains of *Erwinia amylovora,* thereby enhancing the efficacy and reliability of phage-based control strategies.

As we delve deeper into the realm of bacteriophage research and its practical implications, it becomes evident that these tiny viral predators hold great promise in revolutionizing plant disease management strategies. The integration of bacteriophages as sustainable tools to combat *Erwinia amylovora* infections could pave the way for a more environmentally friendly and economically viable approach, mitigating the devastating impact of fire blight on fruit production and preserving the health of our orchards and landscapes.

KR10-2023-0087771 and KR10-2023-0087770 A relate to a bacteriophage having a deposit number KACC 97040P, wherein the bacteriophage exhibits a bacteriophage specific to *Erwinia amylovora* (Erwinia amylovora) and thus has a control effect on fruit burn diseases, The invention can be utilized as a microbial pesticide for controlling fruit burn diseases.

CN117363583A discloses an *Erwinia amylovora* bacteriophage, a preparation and application thereof, wherein the Erwinia amylovora bacteriophage is named as bacteriophage EaP, the preservation number is CCTCC NO: M2022844. The phage EaP provided by the invention has strong host specificity for the lysis of the *Erwinia amylovora* phage, and can realize pollution-free control of plant bacterial disease pear fire blight. KR10-2025403 B1 teaches a bacteriophage named EaP-8 effective for fire blight bacteria and use thereof. The bacteriophage have accession number KFCC11769P and having a lytic activity specific to *Erwinia amylovora* and allegedly have excellent acid resistance, heat resistance, and UV resistance

KR10-2023-0172817 A relates to bacteriophage mixture (Accession No.: KACC 97042) FireFighter-A comprising an Erwinia phage Fifi451, an Erwinia phage Fifi318, an Erwinia phage pEa_SNUABM_27, and an Erwinia phage pEa_SNUABM_47 having lytic activity against at least one microorganism selected from the group consisting of *Erwinia amylovora* and *Erwinia pyrifoliae*. Apparently the mixture is deposited, however not each phage is deposited according to the Budapest treaty.

KR10-2023-0155980 A relates to a bacteriophage comprising any one or more selected from the group consisting of NCBI accession number: MW349138 (pEa_SNUABM_27), NCBI accession number: MZ443773 (pEa_SNUABM_31), NCBI accession number: MZ443774 (pEa_SNUABM_32), NCBI accession number: MT939487 (pEa_SNUABM_47), andpEa_SNUABM_48: NCBI accession number (MW879340), wherein the bacteriophage has killing activity against *Erwinia amylovora* (Erwinia amylovora) and *Erwinia pyrifoliae* (Erwinia pyrifolia).

KR10-2022-0090446 A relates to several bacteriophages and also a cocktail PEp_ SNUABM_ 03 (NCBI accession number: MT822284), pEp_ SNUABM_ 04 (NCBI accession number: MT822285), pEp_ SNUABM_ 08 (NCBI accession number: NB 184886), pEp_ SNUABM_ 11 (NCBI accession number: MT822287) and pEp_ SNUABM_ 12 (NCBI accession number: MT822288), and has an ability to simultaneously kill *Erwinia amylovora* (Erwinia amylovora) and *Erwinia pyrifoliae* (Erwinia pyrifoliae). teaches a bacteriophage, deposit number KACC 97038P, having a lytic activity specific for at least one bacterium selected from the group consisting of *Erwinia amylovora* and *Erwinia pyrifolia*; preferably the the bacteriophage has a lytic activity specific to *Erwinia amylovora.* Preferably the bacteriophage has a nucleotide sequence of SEQ ID NO: 1,

Similar concepts with other phages are disclosed in KR10-2023-0171294 A, KR10-2023-0171303 A, KR10-2023-0171294 A and KR10-2023-0171302 A, wherein the bacteriophages are e.g. in the family Myoviridae or Podoviridae.

KR10-1601912 B1 relates to the bacteriophage, phi EP14 (deposit number KACC 97010), has a lysis ability with respect to *Erwinia pyrifoliae, E. amylovora, E. persicina, E. pisidii, Erwinia sp.,* and *Pantoea stewartii subsp. stewartii* and a gene sequence of seq ID no: 1.

Gill J. J. et al. describe fifty bacteriophage isolates of Erwinia amylovora, the causal agent of fire blight. The authors characterized their phages from several aspects. However, they have observed that the characteristic which did not show any consistent correlation with any of the above phenotypes was host range. This characteristic could not be used to place individual phage into any a specific RFLP group. This lack of association of host range with any other characteristic could very likely be the reason for the great diversity of phages isolated in this study. This fact also suggest that host range cannot be deduced from analysis of the phages and is still a matter of serendipity. [Gill, J. J. et al. Bacteriophages of Erwinia amylovora, Applied and Environmental Microbiology, Apr. 2003, p. 2133-2138]

Boulé et al. collected nineteen active bacteriophages against Erwinia amylovora. Only eight survived the isolation, purification and storage processes and five bacteriophage isolates included in this study lysed more than 50% of the 20 E. amylovora strains tested from BC. Phages_Ea1337-26 and _Ea2345-6 reduced infection by 84% and 96%, respectively, when tested on detached pear blossoms, and bacteriophage _Ea2345-6, applied in combination with Eh21-5, reduced infection of fire blight on apple flowers of potted apple trees by 56% and compared well with the antibiotic streptomycin. [Boulé et al., Isolation and characterization of eight bacteriophages infecting Erwinia amylovora and their potential as biological control agents in British Columbia, Canada, Can. J. Plant Pathol. (2011), 33(3): 308-317]

Knecht LE, et al. deal with phage resistance of *Erwinia amylovora.* Exposing *Erwinia amylovora* to the different phages led to transient resistance which has been analized, as the nature of resistance can differ depending on the phage. The authors propose that EAMY_2231 plays a crucial role in mediating resistance or reduced infectivity against multiple phages simultaneously. This emphasizes the importance of strategies to avoid resistance. [Knecht LE, Born Y, Pelludat C, Pothier JF, Smits THM, Loessner MJ and Fieseler L (2022) Spontaneous Resistance of Erwinia amylovora Against Bacteriophage Y2 Affects Infectivity of Multiple Phages. Front. Microbiol. 13:908346. ]

Thompson, Daniel W. et al. compared 60 completely sequenced bacteriophages that infect Erwinia and/or Pantoea bacteria. The bacteriophage phiEaP-8 referred to carries a 75,929 bp genomic DNA (GenBank accession number, MH160392) which is identical to the phages disclosed by Park J et al., below. [Thompson, Daniel W. et al. Genomic comparison of 60 completely sequenced bacteriophages that infect Erwinia and/or Pantoea bacteria, Virology 535 (2019) 59-73]

Park J. et al. disclose bacteriophage phiEaP-8 which carries a 75,929 bp genomic DNA (GenBank accession number MH160392, [Park J, Lee GM, Kim D, Park DH, Oh CS. Characterization of the Lytic Bacteriophage phiEaP-8 Effective against Both Erwinia amylovora and Erwinia pyrifoliae Causing Severe Diseases in Apple and Pear. Plant Pathol J. 2018 Oct;34(5):445-450. Epub 2018 Oct 1.]

Gayder, S., et al. review the biological control of Erwinia amylovora using bacteriophages from several aspects. The authors admit that field trial data demonstrating the efficacy of phages in fire blight control, to optimize inoculation and application parameters, and to study the effectiveness of a particular formulation is still scarce and the actual effectiveness of the phages is thus often questionable.

In respect of phage resistance the authors note that phages evolved to overcome phage resistance by changing receptor specificities. CRISPR/Cas systems of the host can be rendered inactive by phage encoded anti-CRISPR proteins that inhibit Cas endonucleases. According to the inventors the largely unique genome of Ea PF 8 is advantageuos, because its application in a bacteriophage cocktail can reduce the probability of emerging of a CRISPR-Cas-based immunity in the host.

This suggests that despite the abundance of the phages isolated, there is still a room for actual products and this possibly the concept of claiming protection for novel preparations.

[Gayder, S., Kammerecker, S. & Fieseler, L. Biological control of the fire blight pathogen Erwinia amylovora using bacteriophages. J Plant Pathol (2023).]

Biosca EG et al. claim that they provide the first European *E. amylovora* phage cocktails effective in plant material and suggest that environmental microorganisms can offer effective and sustainable natural solutions for the biocontrol of phytopathogenic bacteria to provide safe and healthy food. The authors acknowledge that their study sets up merely preliminary steps towards designing bacteriophage cocktails effective against fire blight disease in the field and further work is still required to assess their biocontrol capacity under greenhouse and field conditions. [Biosca EG, Delgado Santander R, Morán F, Figàs-Segura À, Vázquez R, Català-Senent JF, Alvarez B. First European Erwinia amylovora Lytic Bacteriophage Cocktails Effective in the Host: Characterization and Prospects for Fire Blight Biocontrol. Biology. 2024; 13(3):176]

Erwinia amylovora continues to be a major threat to Rosaceae plants including a wide range of fruit bearing plants essential in the Agriculture and food-supply. Therefore, there is a huge demand for new and environmentally safe control methods including biopesticide preparations comprising bacteriophages against Erwinia amylovora.

BRIEF DESCRIPTION OF THE INVENTION

*Bacteriophages*

The invention relates to the following bacteriophages capable of infecting and/or lysing *Erwinia amylovora.* Bacteriophage (Ea PF1) deposited in the DSMZ Deutsche Sammlung von Mikroorganismen und Zellkulturen GmbH Inhoffenstr. 7 B38124 Braunschweig GERMANY on March 6, 2024 under accession number DSM 34958 by Environvest Ltd., Pécs, Hungary (bacteriophage DSM 34958). In particular the bacteriophage has SEQ ID NO: 1. The invention also relates to a derivative, variant or mutant of bacteriophage DSM 34958 having a genomic sequence at least 90%, more preferably at least 93% or 95%, even more preferably at least 96%, 97%, 98% or 99% identical to the sequence of SEQ ID NO: 1.

Bacteriophage (Ea PF2) deposited in the DSMZ Deutsche Sammlung von Mikroorganismen und Zellkulturen GmbH Inhoffenstr. 7 B38124 Braunschweig GERMANY on March 6, 2024 under accession number DSM 34958 by Environvest Ltd., Pécs, Hungary (bacteriophage DSM 34959). In particular the bacteriophage has SEQ ID NO: 2. The invention also relates to a derivative, variant or mutant of bacteriophage DSM 34959 having a genomic sequence at least 90%, more preferably at least 93% or 95%, even more preferably at least 96%, 97%, 98% or 99% identical to the sequence of SEQ ID NO: 2.

Bacteriophage (Ea PF3) deposited in the DSMZ Deutsche Sammlung von Mikroorganismen und Zellkulturen GmbH Inhoffenstr. 7 B38124 Braunschweig GERMANY on March 6, 2024 under accession number DSM 34958 by Environvest Ltd., Pécs, Hungary (bacteriophage DSM 34960). In particular the bacteriophage has SEQ ID NO: 3. The invention also relates to a derivative, variant or mutant of bacteriophage DSM 34960 having a genomic sequence at least 90%, more preferably at least 93% or 95%, even more preferably at least 96%, 97%, 98% or 99% identical to the sequence of SEQ ID NO: 3.

Bacteriophage (Ea PF8) deposited in the DSMZ Deutsche Sammlung von Mikroorganismen und Zellkulturen GmbH Inhoffenstr. 7 B38124 Braunschweig GERMANY on March 6, 2024 under accession number DSM 34958 by Environvest Ltd., Pécs, Hungary (bacteriophage DSM 34961). In particular the bacteriophage has SEQ ID NO: 4. The invention also relates to a derivative, variant or mutant of bacteriophage DSM 34961 having a genomic sequence at least 80%, preferably at least 85%, more preferably at least 90% or 95%, even more preferably at least 96%, 97%, 98% or 99% identical to the sequence of SEQ ID NO: 4.

In the present invention each derivative, variant or mutant is capable of infecting or lysing *Erwinia amylovora,* in particular more than one, preferably at least 2, 5, 10, 20 or at least 50% of the *Erwinia amylovora* strains in Table 1 or Table 4.

*Biopesticide compositions*

The invention relates to a biopesticide composition comprising

a) at least one bacteriophage capable of infecting or lysing Erwinia sp bacteria,
b) an UV protectant compound, and
c) a surfactant compound and optionally
d) further excipient compounds.

In a preferred embodiment in the biopesticide composition

a) the at least one bacteriophage capable of infecting or lysing Erwinia sp bacteria is effective against *Erwinia amylovora,* preferably effectively lyses more than one *Erwinia amylovora* strains,
b) the UV protectant compound is an aminobenzoate UV-protectant, and
c) the surfactant compound is a polysaccharide compound, preferably an alkyl polysaccharide.

Preferably the biopesticide compositions comprise on or more of the bacteriophages of the invention (see section Bacteriophages).

The invention also relates to a biopesticide composition (preferably a biopesticide composition according to those comprising the UV-protectant and the surfactant), wherein the one or more bacteriophage is/are capable of infecting or lysing *Erwinia amylovora,*

said bacteriophage selected from the group consisting of

bacteriophage (Ea PF1) deposited under accession number DSM 34958, or a derivative, variant or mutant thereof with a genomic sequence at least 90% identical to the sequence of SEQ ID NO: 1;
bacteriophage (Ea PF2) deposited accession number DSM 34959, or a derivative, variant or mutant thereof with a genomic sequence at least 90% identical to the sequence of SEQ ID NO: 2;
bacteriophage (Ea PF3) deposited under accession number DSM 34960, or a derivative, variant or mutant thereof with a genomic sequence at least 90% identical to the sequence of SEQ ID NO: 3;
bacteriophage (Ea PF8) deposited under accession number DSM 34961, or a derivative, variant or mutant thereof with a genomic sequence at least 80% identical to the sequence of SEQ ID NO: 4;
or any derivative, variant or mutant being identical to the respective sequence to the extent as given in the *Bacteriphages* section above (preferred identity percentages are given above),
each derivative, variant or mutant being capable of infecting/lysing *Erwinia amylovora,* preferably as disclosed herein, and an auxiliary material, preferably an agricultural excipient, wherein preferably
the biopesticide composition comprises more than one isolated bacteriophage capable of infecting or lysing *Erwinia amylovora.*

Preferably the biopesticide composition comprises more than one isolated bacteriophage capable of infecting/lysing *Erwinia amylovora.*

Preferably the invention relates to the biopesticide composition comprising

bacteriophage (Ea PF1) under accession number DSM 34958, or a derivative, variant or mutant thereof with a genomic sequence at least 90% identical to the sequence of SEQ ID NO: 1;
bacteriophage (Ea PF2) under accession number DSM 34959, or a derivative, variant or mutant thereof with a genomic sequence at least 90% identical to the sequence of SEQ ID NO: 2;
bacteriophage (Ea PF3) under accession number DSM 34960, or a derivative, variant or mutant thereof with a genomic sequence at least 90% identical to the sequence of SEQ ID NO: 3;
bacteriophage (Ea PF8) under accession number DSM 34961, or a derivative, variant or mutant thereof with a genomic sequence at least 80% identical to the sequence of SEQ ID NO: 4;
each derivative, variant or mutant being capable of infecting/lysing *Erwinia amylovora,* wherein preferred identity percentages are given above in section Bacteriophages.

Preferably the biopesticide composition of the invention, preferably the composition comprising on or more of the bacteriophages as defined in the Bacteriophage section above, preferably in the previous paragraph, is capable of lysing at least one, preferably at least 2, more preferably at least 10 of the following *Erwinia amylovora* strains listed in table 1 or table 4, and/or,

effective against an *Erwinia amylovora* strain selected from
*Erwinia amylovora* CFBP 1430 = DSM 34956
*Erwinia amylovora* OR-25 = DSM 34957, and/or
capable of lysing at least one, preferably at least 2, more preferably at least 10 of the following *Erwinia amylovora* strains listed in table 2.

Preferably

- phage Ea PF 1 (DSM 34958) or its derivative, variant or mutant is capable of lysis of at least 90% of bacteria of the Ea. strains of table 4, preferably confluent lysis of at least 50%, preferably at least 70 % of the Ea. strains of table 4,
- phage Ea PF 2 (DSM 34959) or its derivative, variant or mutant is capable of lysis of at least 50%, preferably at least 60 % of the Ea. strains of table 4, preferably confluent lysis of at least 25% of the Ea. strains of table 4,
- phage Ea PF 3 (DSM 34960) or its derivative, variant or mutant is capable of lysis of at least 60%, preferably at least 70 % of the Ea. strains of table 4, preferably confluent lysis of at least 25% of the Ea. strains of table 4,
- phage Ea PF 8 (DSM 34961) or its derivative, variant or mutant is capable of lysis of at least 90%, preferably 95%, highly preferably 100% of bacteria, preferably confluent lysis of at least 30%, preferably at least 35 % of the Ea. strains of table 4,

with spot test.
Preferably

- phage Ea PF 1 (DSM 34958) or its derivative, variant or mutant is capable to produce at least $10^{11}$ genome copies/ml in the case of at least 60%, preferably in 73% of the tested bacteria,
- phage Ea PF 2 (DSM 34959) or its derivative, variant or mutant is capable to produce at least $10^{11}$ genome copies/ml in the case of at least 12%, preferably in 16% of the tested bacteria,
- phage Ea PF 3 (DSM 34960) or its derivative, variant or mutant is capable to produce at least $10^{11}$ genome copies/ml in the case of at least 12%, preferably in 16% of the tested bacteria,
- phage Ea PF 8 (DSM 34961) or its derivative, variant or mutant is capable to produce at least $10^{11}$ genome copies/ml in the case of at least 60%, preferably in 70% of the tested bacteria,

if measured by QPCR. (Preferred identity percentages are given above.)
Preferably each of the phages are heat tolerant up to 50°C. Preferably Ea PF1 and/or Ea PF8 are heat tolerant up to 60°C.
Preferably Ea PF1 and/or Ea PF8 are tolerant to desiccation during 24 hours.

*Cocktail of bacteriophages*

The invention also relates to a cocktail of bacteriophages comprising two or more bacteriophages selected from the bacteriophages as defined in the *Bacteriophages* section and a biologically, preferably agronomically tolerable excipient.
The invention also relates to a cocktail of bacteriophages comprising two or more bacteriophages selected from the bacteriophages as defined in the *Biopesticide compositions* section and a biologically, preferably agronomically tolerable excipient.

Preferably the cocktail of bacteriophages also comprises

b) an UV protectant compound, and
c) a surfactant compound and optionally
d) further excipient compounds.

In a preferred embodiment

b) the UV protectant compound is an aminobenzoate UV-protectant, and
c) the surfactant compound is a polysaccharide compound, preferably an alkyl polysaccharide.

Preferably the cocktail of bacteriophages comprises

bacteriophage (Ea PF1) deposited under accession number DSM 34958, or a derivative, variant or mutant thereof with a genomic sequence at least 90% identical to the sequence of SEQ ID NO: 1;
bacteriophage (Ea PF2) deposited under accession number DSM 34959, or a derivative, variant or mutant thereof with a genomic sequence at least 90% identical to the sequence of SEQ ID NO: 2;
bacteriophage (Ea PF3) deposited under accession number DSM 34960, or a derivative, variant or mutant thereof with a genomic sequence at least 90% identical to the sequence of SEQ ID NO: 3; and
bacteriophage (Ea PF8) deposited under accession number DSM 34961, or a derivative, variant or mutant thereof with a genomic sequence at least 80% identical to the sequence of SEQ ID NO: 4;
each derivative, variant or mutant being capable of infecting *Erwinia amylovora* as disclosed herein e.g. in above paragraphs. (Preferred identity percentages are given above.)

*Isolated bacteriophage or cocktails*

The invention also relates to an isolated bacteriophage as defined in the section Bacteriophages or the cocktail of isolated bacteriophages.

In particular, the invention relates to an isolated bacteriophage or the cocktail of isolated bacteriophages capable of infecting/lysing *Erwinia amylovora* said bacteriophage selected from the group consisting of Bacteriophage (Ea PF8) deposited in the DSMZ Deutsche Sammlung von Mikroorganismen und Zellkulturen GmbH Inhoffenstr. 7 B38124 Braunschweig GERMANY on March 6, 2024 under accession number DSM 34958 by Environvest Ltd., Pécs, Hungary (bacteriophage DSM 34961). In particular the bacteriophage has SEQ ID NO: 4. The invention also relates to a derivative, variant or mutant of bacteriophage DSM 34961 having a genomic sequence at least 80%, preferably at least 85%, more preferably at least 90% or 95%, even more preferably at least 96%, 97%, 98% or 99% identical to the sequence of SEQ ID NO: 4.

Preferably said isolated bacteriophage Ea PF8 is capable of lysing activity on at least 90% of the *Erwinia amylovora* strains listed in Table 4.

Preferably confluent lysis (4+) is present in at least 30, preferably at least 40% of the bacteria.

Preferably the Ea PF 8 phage is able to produce $10^{11}$ genome copies/ml in the case of 70% of the tested bacteria as shown by QPCR.

Preferably the Ea PF 8 phage is effective and can produce more than $10^9$ genome copies/ml in 67% of tested bacteria strains in Table 2.

Preferably the Ea PF 8 phage is heat tolerant, preferably tolerant at 50°C for at least 1 minute, preferably at least 2 minutes, preferably according to the measurement described in the materials and methods.

Preferably the Ea PF 8 phage is desiccation tolerant for at least 24 hours.

*Uses*

The invention also relates to a use of the isolated bacteriophage or the cocktail as defined in any of the above for the control of *Erwinia amylovora.*

Preferably the use comprises application of the isolated bacteriophage on a plant, preferably a Rosaceae plant, to prevent or treat bacterial infection caused by *Erwinia amylovora.*

The invention also relates to a use of the biopesticide composition as defined in the *biopesticide composition* section for the control of *Erwinia amylovora.*

Preferably the use comprises application of the biopesticide composition to a plant, preferably a Rosaceae plant, to prevent or treat bacterial infection caused by *Erwinia amylovora.*

*Methods*

The invention also relates to a method for the control of *Erwinia amylovora,* comprising the steps of application of a bacteriophage according to any of phages defined herein or above, in particular in the *Bacteriophages* section or in the *Biopesticide compositions* section on a plant preferably a Rosaceae plant, to prevent or treat bacterial infection caused by *Erwinia amylovora.*

Preferably the invention relates to a method to prevent or treat bacterial infection caused by *Erwinia amylovora* in a plant, preferably in a Rosaceae plant, said method comprising the step of contacting the plant with the biopesticide composition as defined above.

Preferably in the method of the invention the flowers, leaves or shoots of the plant are contacted, depending on the status of the plant.

Preferably the method comprises applying the biopesticide composition on the flowers, leaves and/or shoots of the plant multiple times a year.

Preferably in the method of the invention the flowers, the leaves or the shoots of the plant are contacted at least one time, or preferably

at least two times within 30 days, or preferably
at least three times within 20 days.

Preferably in the uses and methods of the invention the Rosaceae plant is selected from the group consisting of apples, pears, quinces, medlars, loquats, almonds, peaches, apricots, plums, cherries, strawberries, blackberries, raspberries, sloes, and roses.

Preferably the Rosaceae plant is selected from the group consisting of apples, pears, quinces, medlars, almonds, peaches, apricots, plums and cherries, particularly preferably apples and pears, hignly preferably apples. Preferably the Rosaceae plant is selected from the subfamilies *Amygdaloideae, Rosoideae* and *Dryadoideae,* preferably selected from *Amygdaloideae* and *Rosoideae.*

Highly preferably the plant is a fruit tree plant of the Rosaceae family, preferably said plant is selected from apples, pears and quince, highly preferably apples.

DEFINITIONS

A *"bacteriophage"* or briefly a "phage" is a virus capable of infecting a bacterium.

A *"lytic phage"* is a bacteriophage that reproduces according to a lytic cycle. In the lytic cycle, the DNA of the virus is replicated separately from the DNA of the host cell during viral replication. During the lytic cycle, the infected bacterial cell breaks down and dies (lysis occurs). Phages that exclusively use the lytic cycle are also called virulent phages.

A bacteriophage *"derivative"* is a bacteriophage progeny produced by culturing, multiplying or growing a given bacteriophage. The derivative has genetically identical parts with or essentially identical to the parent bacteriophage. The level of identity with the parent phage can be given in percentages.

A *"variant"* of a bacteriophage means a subtype of a bacteriophage that is genetically distinct from that bacteriophage, but that does not differ sufficiently in characteristics to be considered a separate bacteriophage, i.e. typically has a highly similar genome and, as used herein, has essentially identical effect.

A "mutant" bacteriophage is a bacteriophage that contains one or more mutations in its genome compared to the genome of the parent bacteriophage. A mutant usually loses or gains one or more traits or characteristics compared to the parent bacteriophage and nevertheless its genome is highly similar to the parent bacteriophage. A *"composition"* as used herein is a composition of matter comprising an active agent and an auxiliary material. The active agent is capable of exerting a biological effect whereas the auxiliary material is useful in formulating the composition or to provide appropriate environment to the active agent to allow exerting its effect. Preferably the auxiliary material is an UV-protectant and/or a surfactant.

An *"agricultural composition "* is a composition useful in any area of agriculture, in application of pest control in plants.

A *"biopesticide composition"* is a biological substance or organism that damages, kills, or repels organisms seen as pests whereas leaves intact others which are useful or are to be protected. In an example the pest is a biological pest of a plant. Biological pest management intervention involves application of bacteriophages on plants.

*"Capable of infecting Erwinia amylovora"* is understood herein as being capable of infecting/lysing at least one *Erwinia amylovora* strain, preferably at least one, at least two or preferably at least n *Erwinia amylovora* strain selected from the group consisting of the strains listed in Table 1 or Table 4 and preferably

*Erwinia amylovora* CFBP 1430 = DSM 34956

*Erwinia amylovora* OR-25 = DSM 34957

*"Heat tolerance"* is understood herein as a measure of the capability of the bacteriophages to withstand heat; "Heat tolerance" is typically measured at a given temperature for a time by testing the capability of the phages to infect their host. For example, heat tolerance can be measured by a spot test. For example the number of phages capable of infecting is measured initially ($N_0$) and at a given time (Nt) and ratio is calculated; provided that this ratio is within a threshold level the phage can be considered as heat tolerant. In a variant log of the ratio of ($N_0/N_t$) is less than 1.5, preferably less than 1, preferably less than 0.5.

*"Desiccation tolerance"* is understood herein as a measure of the capability of the bacteriophages to withstand drought or dryness; "Desiccation tolerance" is typically measured at given dry conditions temperature for a time by testing the capability of the phages to infect their host. For example, desiccation tolerance can be measured by a spot test. For example the number of phages capable of infecting is measured initially ($N_0$) and at a given time ($N_t$) and ratio is calculated; provided that this ratio is within a threshold level the phage can be considered as heat tolerant. In a variant log of the ratio of ($N_0/N_t$) is less than 2, preferably is less than 1.5, preferably less than 1, preferably less than 0.5.

The singular forms *"a", "an"* and *"the",* or at least *"a", "an",* include plural reference unless the context clearly dictates otherwise.

The term *"comprises"* or *"comprising"* or *"including"* are to be construed here as having a non- exhaustive meaning and allow the addition or involvement of further features or method steps or compo-nents to anything which comprises the listed features or method steps or components. *"Comprising"* can be substituted by *"including"* if the practice of a given language variant so requires or can be limited to *"consisting essentially of"* if other members or components are not essential to reduce the invention to practice or to *"consisting of"*.

BRIEF DESCRIPTION OF THE FIGURES

Figure.1 Map of the plasmid pQSTD2.17 used in quantitative PCR reactions.
Figure 2a Genome map of the phage Ea PF 8.
Figure 2b Phylogenetic tree of the phage Ea PF8
Figure 3. a. Desiccation tolerance of Ea PF8. b. Temperature stability of Ea PF8
Figure 4 Phylogenetic trees of phages in the cocktail
Figure 5. Heat tolerance of phages in the cocktail at 50°C and 60°C 5a: Ea PF2, 5b: Ea PF3, 5c: Ea PF8, 5d: Ea PF1.
Figure 6. Desiccation tolerance of phages in the cocktail during 24 hours.
Figure 7. Effect of the additives on the phage Infectivity.
Figure 8. Effect of the additives against UV irradiation.
Figure 9. Green house experiment results for 10 and 20 days of monitoring, a. positive control of infection. b. Effect of the "New cocktail" on infection. c. Effect of the "Old cocktail" on infection. d.. Effect of the Serenade on infection
Figure 10. Products efficacy in control infections with fire blight on Topaz apple trees, on blossoms, shoots and fruits - *Erwinia amylovora* inside the experimental plot Research Institute for Fruit Growing Piteşti Romania, N 44.89'99,15"; E 24.86'33,10"; Alt. 273 m a.s.l.

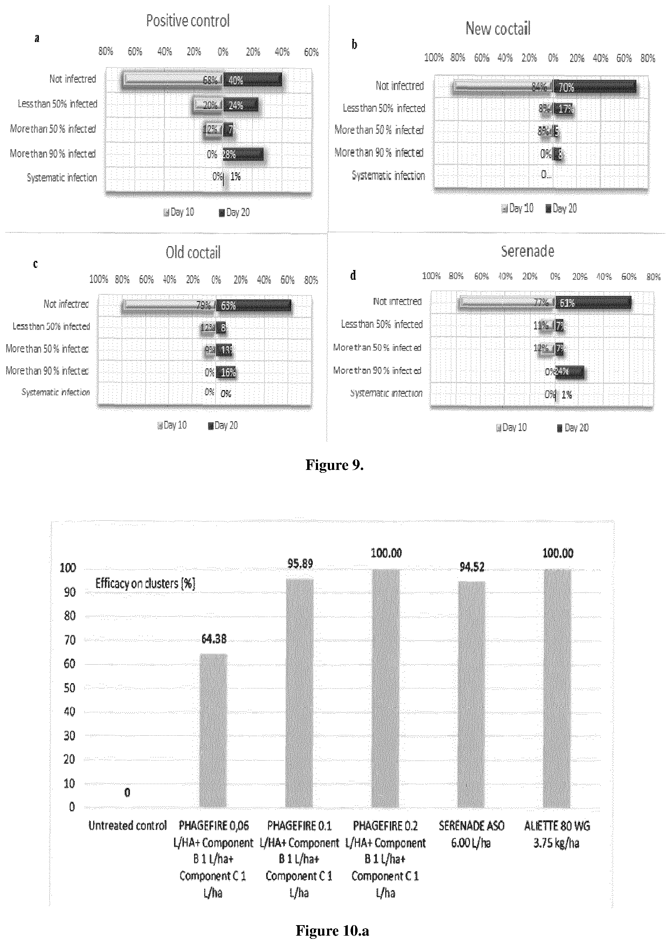

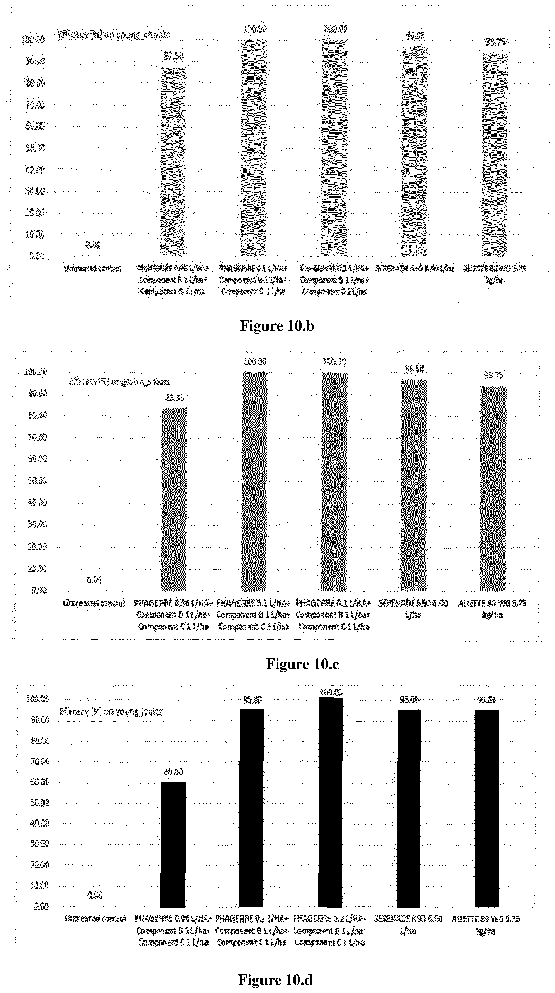

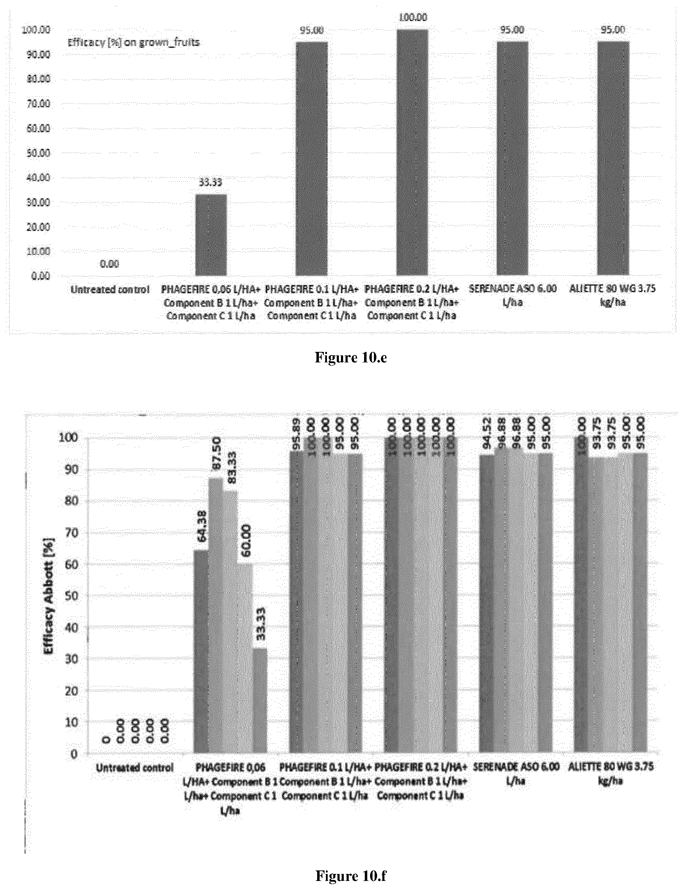

Figure 10.a Product efficacy in control of fire blight - Erwinia amylovora on 'Topaz' variety flower clusters.
Figure 10.b Product efficacy in control of fire blight - Erwinia amylovora on 'Topaz' apple young shoots.
Figure 10.c Product efficacy in control of fire blight - Erwinia amylovora on 'Topaz' apple grown shoots.
Figure 10.d Product efficacy in control of fire blight - Erwinia amylovora on 'Topaz' apple young fruits.
Figure 10.e Product efficacy in control of fire blight - Erwinia amylovora on 'Topaz' apple grown fruits.
Figure 10.f Overall efficacy of PHAGEFIRE in protecting "Topaz" apple trees on spurs, shoots and fruits against fire blight.

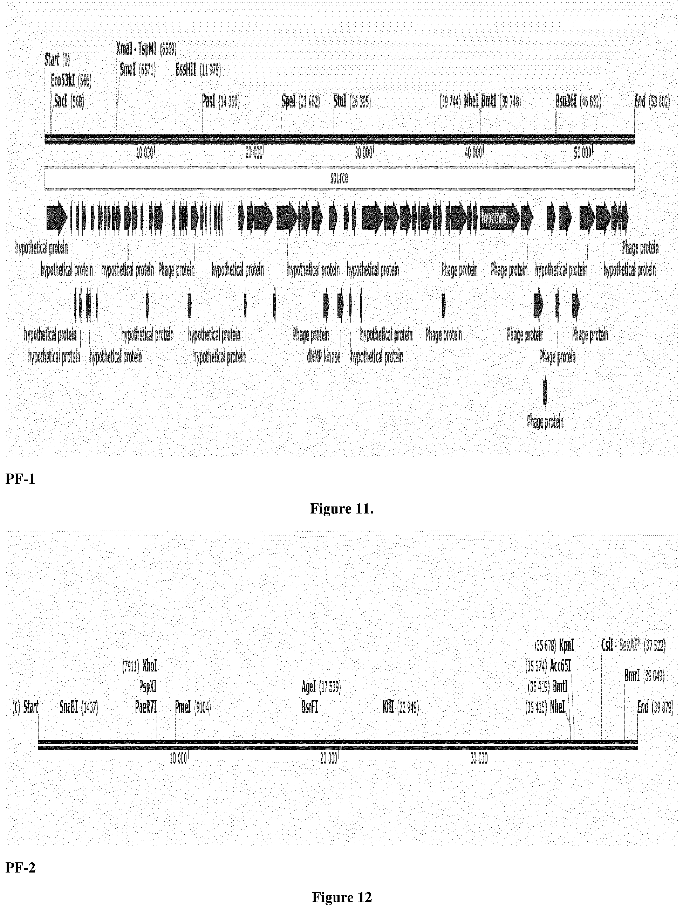

Figure 11. Total genomic map of Bacteriophage infecting *Erwinia amylovora* Ea PF 1 (DSM 34958)
Figure 12. Total genomic map of Bacteriophage infecting *Erwinia amylovora* Ea PF 2 (DSM 34959)

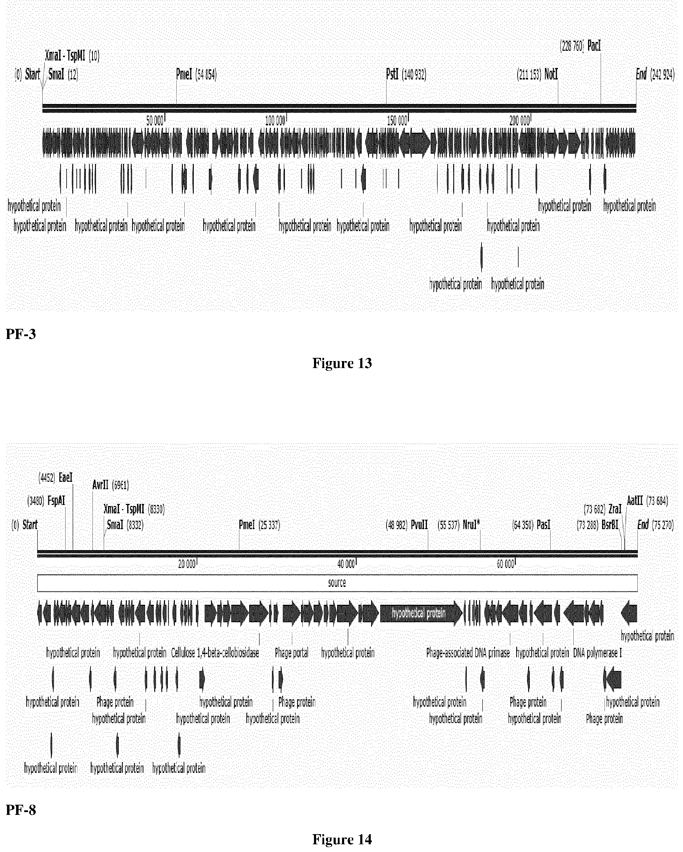

Figure 13. Total genomic map of Bacteriophage infecting *Erwinia amylovora* Ea PF 3 (DSM 34960)
Figure 14. Total genomic map of Bacteriophage infecting *Erwinia amylovora* Ea PF 8 (DSM 34961)

DETAILED DESCRIPTION OF THE INVENTION

Fire blight caused by *Erwinia amylovora* is one of the most damaging disease in fruit growing countries, for fruits production and for healthy planting material and new pome and stone orchards establishment as well. Attempts to fight against fire blight using healthy biologic material, biological products, chemical ones, different application timing and rates upon a well-defined protocols and good agricultural practices is more than necessary. The inventors made significant effort to find a bacteriophage control against this damaging disease. While bacteriophage-based biopesticide composition promise an environment friendly tool against fire-blight at present the need remains to find such a control method.

In the present invention phages were isolated from apple orchards, which was collected in Romania, Hungary and Spain. Phages was isolated according to method which is provided by Gill et.al. [Gill, J. J. et al. Bacteriophages of Erwinia amylovora, Applied and Environmental Microbiology, Apr. 2003, p. 2133-2138]. Phage detection, Purification and identification of the phages were done with a combination of spot test, double agar overlay, and qPCR methods as known in the art.

To develop of phage product with a wide activity spectrum sampling was done in different countries: Spain, Hungary, and Romania. Samples were taken from infected apple orchards. After isolation and purification samples were sequenced to determine different ones, and selection was made based on host range, receptor specificity of isolated phages. Surprisingly, a few phages of outstanding properties were found, in particular phages PF-1 and PF-8, particularly preferably PF-8. For the cocktail, formulations were chosen for 4 phages with different receptor and host range specificity: PF-1, PF2, PF-3 and PF-8.

Unexpectedly, it has been noticed that a combination of these phages has a high efficacy and a very broad spectrum of host.

*Erwinia amylovora* bacteriophage Ea PF1 has been deposited in the DSMZ Deutsche Sammlung von Mikroorganismen und Zellkulturen GmbH Inhoffenstr. 7 B38124 Braunschweig GERMANY on March 6, 2024 under accession number DSM 34958 by Environvest Ltd., Pécs, Hungary (bacteriophage DSM 34958). The genome of the bacteriophage has been sequenced and its sequence is shown on SEQ ID NO: 1.

*Erwinia amylovora* bacteriophage Ea PF2 has been deposited in the DSMZ Deutsche Sammlung von Mikroorganismen und Zellkulturen GmbH Inhoffenstr. 7 B38124 Braunschweig GERMANY on March 6, 2024 under accession number DSM 34959 by Environvest Ltd., Pécs, Hungary (bacteriophage DSM 34959). The genome of the bacteriophage has been sequenced and its sequence is shown on SEQ ID NO: 2.

*Erwinia amylovora* bacteriophage Ea PF3 has been deposited in the DSMZ Deutsche Sammlung von Mikroorganismen und Zellkulturen GmbH Inhoffenstr. 7 B38124 Braunschweig GERMANY on March 6, 2024 under accession number DSM 34960 by Environvest Ltd., Pécs, Hungary (bacteriophage DSM 34960). The genome of the bacteriophage has been sequenced and its sequence is shown on SEQ ID NO: 3.

*Erwinia amylovora* bacteriophage Ea PF8 has been deposited in the DSMZ Deutsche Sammlung von Mikroorganismen und Zellkulturen GmbH Inhoffenstr. 7 B38124 Braunschweig GERMANY on March 6, 2024 under accession number DSM 34961 by Environvest Ltd., Pécs, Hungary (bacteriophage DSM 34961). The genome of the bacteriophage has been sequenced and its sequence is shown on SEQ ID NO: 3.

There are several factors that can affect phages in an open environment, such as sunlight, and desiccation heat. To ensure UV protection and effective spreading of the phages, in the product were added UV protectant and surfactant components. In the particular examples shown below the compositions were formulated by adding commercially available PABA and Atlox AL-2575 (alkyl polysaccharide).

In a particularly preferred embodiment, phage Ea PF 8 was isolated from apple orchards from Hungary, as it was shown from DNA sequence analysis there is some relatedness to other phages, based on the percentage of this relatedness phage Ea PF 8 appears to be a new phage (see the Examples). The phage was tested with Hungarian as well as with other *Erwinia amylovora* strains from worldwide collections.

The results revealed that Ea PF 8 has a surprisingly broad host range, so we can consider it a lytic phage [Born Y, Fieseler L, Marazzi J, Lurz R, Duffy B, Loessner MJ. Novel virulent and broad-host-range Erwinia amylovora bacteriophages reveal a high degree of mosaicism and a relationship to Enterobacteriaceae phages. Appl Environ Microbiol. 2011 Sep;77(17):5945-54.]., which is one of the important features of using phages against bacteria in practice. So, all results obtained during testing phage features such as genome sequence, morphology, effectiveness against target bacteria, and stability towards environmental stress is suggesting strong potential for its future use as an effective and realistic antibacterial agent against fire blight.

Phages are often have a very specific host specificity and are often are non-lytic on their hosts. Thus, finding a lytic phage with high efficiency is an unforeseen task for a person skilled in the art, in particular if a specific phage with a unique genome is to be found.

P8 bacteriophage has a unique genome. This is advantageous, because its application in a bacteriophage cocktail can reduce the probability of emerging of a CRISPR-Cas-based immunity in the host.

Bacteriophage cocktails containing several phages have been developed to counteract the emergence of bacterial resistance [Kim, H.J.; Jun, J.W.; Giri, S.S.; Kim, S.G.; Kim, S.W.; Kwon, J.; Lee, S.B.; Chi, C.; Park, S.C. Bacteriophage cocktail for the prevention of multiple-antibiotic-resistant and mono-phage-resistant Vibrio coralliilyticus infection in pacific oyster (Crassostrea gigas) larvae. Pathogens 2020, 9, 831. 24. Kim, S.G.; Lee, S.B.; Jo, S.J.; Cho, K.; Park, J.K.; Kwon, J.; Giri, S.S.; Kim, S.W.; Kang, J.W.; Jung, W.J.; et al. Phage cocktail in combination with kasugamycin as a potential treatment for fire blight caused by Erwinia amylovora. Antibiotics 2022, 11, 1566.].

Besides identifying phages with outstanding properties finding a cocktail composition which is effective in practice is a non-obvious task [Biosca EG and Delgado Santander R, et al. First European Erwinia amylovora Lytic Bacteriophage Cocktails Effective in the Host: Characterization and Prospects for Fire Blight Biocontrol. Biology. 2024; 13(3):176]. In particular, a cocktail formulation which is effective, and in particular against a broad range of *Erwinia amylovora* hosts in non obvious. The inventors have prepared cocktail formulations provide a broader spectrum of activity, targeting different strains of *Erwinia amylovora,* thereby enhancing the efficacy and reliability of phage-based control strategies.

The cocktail of the invention has been tested also under greenhouse and field conditions as illustrated in the Examples by several studies.

To mention a particular study, we exemplify herein in the detailed discussion a study carried out in Romania, the right terrace of the Doamnei River on apple cultivar Topaz.

The product of the invention was applied 3 times just before sunset, using an atomiser. Bacteriosis incidence, severity and damages degrees on leaflets, shoots, and fruits were evaluated 6 times, after every application till the end of August in 2023. These local conditions, including meteorology, seemed to be good for fire blight infections and disease development; nevertheless, due the microclimate particularities of this year, during month of August, the infection potential with Erwinia amylovora bacteria was low.

The assessments carried on the studied variety 'Topaz' revealed that it behaved like sensitive according to its genetic background, treatments applied and moment of evaluation.

It has been surprisingly found that the composition of the invention protected very well the 'Topaz' apple blossoms against the early infections with Erwinia amylovora bacteria (Figure 10.a). Moreover, the composition protected very well the 'Topaz' apple shoots against infections with Erwinia amylovora bacteria (Figure 10.b and 10.c). Moreover, the 'Topaz' apple fruits were also fully protected against infections with Erwinia amylovora bacteria with the composition applied at a rate of 0.2 L/ha.

A similar trial was carried out in 2023, in Rincón de Soto (La Rioja), in pear tree crop (*Pyrus communis*), cultivar 'Conferencia', to assess the efficacy and crop safety of the cocktail of the invention in foliar application against fireblight (*Erwinia amylovora*), and also in other sites in Europe (see the Examples).

The composition of the invention had excellent effect under several experiments.

Moreover, no phytotoxicity found up to date by visual assessment from any tested product at any timing in the apple tree crop and no effects on non-target organisms up to date found by visual assessment from any tested product at any timing.

The skilled person will be aware that alternative compositions can be made, for example other UV-protecting agents and binders, like surfactants can be applied.

The skilled person will also be aware that mutants and derivatives of the phages can be prepared. Bacteriophages can be engineered or even prepared by total synthesis.

Already J. Craig Venter group in the beginning of this century have designed method to build up total phage genomes by accurate assembly of 5- to 6-kb segments of DNA from synthetic oligonucleotides. Their methodology was useful for the rapid (14-day) assembly of the complete infectious genome of bacteriophage φX174 (5,386 bp) from a single pool of chemically synthesized oligonucleotides. [Smith, Hamilton O. Clyde A. Hutchison III, Cynthia Pfannkoch and J. Craig Venter PNAS, Vol. 100, No. 26 (Dec. 23, 2003), pp. 15440-15445 (6 pages)].

Huang-Jie Front [Huang-Jie Front. Microbiol., 31 May 2023 Sec. Phage Biology Volume 14 - 2023] gives a review of phage engineering methods, including in-host and out-of-host engineering.

Sun Q, et al. also reviews synthetic biology methods exemplifying phage synthetic biology, and outline the current synthesis and application of engineered phages in practice [Sun Q, et al. Advance on Engineering of Bacteriophages by Synthetic Biology. Infect Drug Resist. 2023;16:1941-1953].

Garenne, David et al. [Garenne, David et al. Current Opinion in Systems Biology, Volume 28, 2021, 100373] report that the cell-free gene expression (CFE) methods have been considerably improved in the last decade. As a consequence, CFE systems have now the capacity to express DNAs composed of tens of genes encoding for complex self-assembly processes. The authors demonstrate that infectious bacteriophages can be synthesized nowadays even in one-pot CFE reactions from their genomes.

Below the invention is further illustrated by non-limiting examples.

EXAMPLES

**EXAMPLE 1**

**Bacterial Strains**

For phage isolation, purification, and characterization we used *Erwinia amylovora* strains from the National Collection of Agricultural and Industrial Microorganisms (Hungary) are listed in Table 1. and with worldwide collection as well in Table 2. Bacterial Strains were grown in LB broth and on LB 1.5 % agar plates.

Table 1.The list of Hungarian *Erwinia amylovora* strains

| Erwinia amylovora | Strain collection | Isolation cource |
|---|---|---|
| Ea B 01272 | National Collection of Agricultural and Industrial Microorganisms (Hungary) | Apple |
| Ea B 01616 | " | Unknown (plant) |
| Ea B 01728 | " | Apple |
| Ea B 01729 | " | Apple |
| Ea B 01731 | " | Apple |
| Ea B 01733 | " | Apple |
| Ea B 01734 | " | Pear |
| Ea B 01735 | " | Quince |
| Ea B 01738 | " | Medlar |
| Ea B 01756 | " | Apple |
| Ea B 01757 | " | Apple |
| Ea B 01840 | " | Crategus sp |
| Ea B 01843 | " | Apple |
| Ea B 01844 | " | Pear |
| Ea B 01853 | " | Cotoneaster sp |
| Ea B 01855 | " | Pyracantha sp |
| Ea B 01896 | " | Unknown (plant) |
| Ea B 01898 | " | Apple |
| Ea B 01901 | " | Apple |
| Ea B 01903 | " | Apple |
| Ea B 01905 | " | Apple |
| Ea B 01906 | " | Apple |
| Ea B 01960 | " | Apple |
| Ea B 01961 | " | Apple |
| Ea B 01962 | " | Apple |
| Ea B 01963 | " | Apple |
| Ea B 01964 | " | Apple |
| Ea B 01971 | " | Apple |
| Ea B 01972 | " | Pear |
| Ea B 01973 | " | Pear |
| Ea B 01975 | " | Pear |
| Ea B 01978 | " | Pear |
| Ea B 01980 | " | Apple |
| Ea B 01983 | " | Quince |
| Ea G254 | Government Office of Baranya Country, Hungary | Apple |
| Ea G255 | Government Office of Baranya Country, Hungary | Apple |
| Ea B0118T | National Collection of Agricultural and Industrial Microorganisms (Hungary) | Pear |

**Table 2.** The list of *Erwinia amylovora* strains from worldwide collections

| Strain | Group | Origin of the Stain |
|---|---|---|
| CFBP 1430 | Amygdaloideae | CIRM-CFBP French Collection for Plant Associated Bacteria |
| 4/82 | Amygdaloideae | P. communis, Egypt, 1982, W. Zeller |
| Ea 56400 | Amygdaloideae | Pyrus sp., Fribourg CH, 2007 |
| 01SFR-BO | Amygdaloideae | Italy, 1991 [41] |
| LA477 | Amygdaloideae | Oregon state university, OR, USA |
| LA071 | Amygdaloideae | " |
| Ea644 | Amygdaloideae | USA [41] |
| Tk119 | Amygdaloideae | Pyrus sp, Izmir, Turkey, 2011 |
| Ea263 | Amygdaloideae | Cydonia so, Israel, 1997. |
| UPN527 | Amygdaloideae | Spain,1996 [41] |
| LebA3 | Amygdaloideae | Malus sp.,Lebanon,1998 |
| SAE3 | Amygdaloideae | Malus sp., Saratov, Russia, 2012 |
| MOE1 | Amygdaloideae | Malus sp., Moldova, 2007 |
| TE4 | Amygdaloideae | Malus sp., Tambov, Russia, 2007 |
| Tk86 | Amygdaloideae | Cydonia sp., Bursa, Turkey,2007 |
| KBE1 | Amygdaloideae | Cydonia sp., Kabardino-Balkaria, Russia, 2009 |
| VGE1 | Amygdaloideae | Cydonia sp., Volgograd, Russia, 2010 |
| Ea2 | Amygdaloideae | Pyrus sp., Lorestan, Iran, 2009 |
| CFBP 1349 | Amygdaloideae | CIRM-CFBP French Collection for Plant Associated Bacteria |
| Ea33 | Amygdaloideae | Cydonia sp., Semnan, Iran,2009 |
| Ea1 | Amygdaloideae | Pyrus sp., Iran, 2009 |
| VRE4 | Amygdaloideae | Malus sp., Voronezh, Russia, 2010 |
| CFBP3098 | Amygdaloideae | CIRM-CFBP French Collection for Plant Associated Bacteria |
| CFBP3860 | Amygdaloideae | " |
| OR29 | Amygdaloideae | Hood River, Oregon state university, OR, USA |
| LebB66 | Amygdaloideae | Cydonia sp., Lebanon, 1998 |
| UTRJ2 | Amygdaloideae | Malus sp,. UT, USA, 2000 |
| CFBP3792 | Amygdaloideae | CIRM-CFBP French Collection for Plant Associated Bacteria |
| TK159 | Amygdaloideae | Cydonia sp., Zonguldak, Turkey, 2012 |
| 87-73 | Amygdaloideae | Malus sp., WA,Usa, |
| BH | Amygdaloideae | Malus sp., MI, USA, 2008 |
| CA3R | Amygdaloideae | Malus sp., Ca, USA, 1995 |
| FB93-9 | Amygdaloideae | Malus sp., ID, USA |
| NW1-1 | Amygdaloideae | Malus sp., MI, USA, 2011 |
| OKR1 | Amygdaloideae | Rubus sp., OK, USA, 2002 |
| RBA4 | Amygdaloideae | Rubus sp., MI, USA |
| RKK3 | Amygdaloideae | Rubus sp., MI, USA |
| TxLo3 | Amygdaloideae | Loquat, TX, Usa,2011 |
| WSDA16 | Amygdaloideae | Washington State Department of Agriculture, USA |
| SE1 | Amygdaloideae | Malus sp., Samara, Russia, 2008 |
| SE11 | Amygdaloideae | Malus sp., Samara, Russia, 2012 |
| FEa9 | Amygdaloideae | Pyracantha sp.,Wojewodztwo Mazowiecki, Poland,2011 |
| KE9 | Amygdaloideae | Crataegus sp.,Kaliningrad, Russia, 2003 |
| Ea-32 | Amygdaloideae | Cydonia sp., East-Azerbaizjan, Iran, 2009 |
| Ea-275 | Amygdaloideae | Cotoneaster sp., Stammheim, CH,1989 |
| CFBP3049 | Amygdaloideae | CIRM-CFBP French Collection for Plant Associated Bacteria |
| Ea646 | Amygdaloideae | Rubus sp., USA |
| Ea625a | Amygdaloideae | Rubis so., USA, 1996 |
| Ea530 | Amygdaloideae | Rubus sp., USA, 1949 |
| Ea592 | Amygdaloideae | Rubus sp., USA, 1995 |

(continued)

| Strain | Group | Origin of the Stain |
|---|---|---|
| 393-3NZ | Amygdaloideae | Malus sp., Nelson, South Island, New Zealand, 1998 |
| 8665NZ | Amygdaloideae | Malus sp., Appleby, South Island, New Zealand, 1984 |
| JL1185 | Amygdaloideae | Pyrus sp., Quincy, WA, USA |
| CFBP1232 | Amygdaloideae | CIRM-CFBP French Collection for Plant Associated Bacteria |
| CFBP2586 | Amygdaloideae | " |
| JL1189 | Amygdaloideae | Pyrus sp., Granger, WA, USA, 1988 |
| IL-5 | Amygdaloideae | Rubus sp., USA, 1977 |
| IH3-1 | Amygdaloideae | Indian Hawthorn, LA, USA, 1998 |
| OR25 | Amygdaloideae | Hood River, Oregon state university, OR, USA |
| UTFer2 | Amygdaloideae | Malus sp., UT, USA, 2000 |
| LA025 | Amygdaloideae | Oregon state university, OR, USA, 1988 |
| LA036 | Amygdaloideae | " |
| LA096 | Amygdaloideae | " |
| LA 102 | Amygdaloideae | " |
| JL1168 | Amygdaloideae | Pyrus sp., Quincy, WA, USA, 1988 |
| JL1170 | Amygdaloideae | Pyrus sp., Quincy, WA, USA, 1988 |
| LA092 | Amygdaloideae | Oregon state university, OR, USA |
| AFRS2 | Amygdaloideae | Malus sp., Alabama, USA, 1994 |
| MO-35 | Amygdaloideae | Moldavia, Malus sp., 2007 |
| MO-E-101b | Amygdaloideae | Moldavia, Malus sp., 2007 |
| SLAPL#3 | Amygdaloideae | Malus sp., Ca, USA, 1995 |
| CFBP2301 | Amygdaloideae | CIRM-CFBP French Collection for Plant Associated Bacteria |
| Ea209 | Amygdaloideae | Agricultural Reasearch oraganization of Israel, Bet-Degan, Israel |
| MR-1 | Amygdaloideae | USA [41] |
| CFBP3020 | Amygdaloideae | CIRM-CFBP French Collection for Plant Associated Bacteria |
| CFBP1252 | Amygdaloideae | " |
| Ea495 | Amygdaloideae | Sorbus sp., Ireland, 1986 |
| Ea266 | Amygdaloideae | Malus sp., Ontario, Canada, 1983 |
| Ea01-03p | Amygdaloideae | Pyrus sp., Nova Scotia, Canada |
| PD2915 | Amygdaloideae | Amelanchier, Canada, 1996 |
| E7004M | Amygdaloideae | BC Canada |
| IL-6 | Amygdaloideae | Rubus sp., USA 1998 |
| CPFB1309 | Amygdaloideae | CIRM-CFBP French Collection for Plant Associated Bacteria |
| 99.12 | Amygdaloideae | Cotoneaster sp., Auckland, Nort Island, New Zealand, 1999 |
| 8862 | Amygdaloideae | Malus sp., Hastings, North Island, New Zealand, 1984 |
| 13300 | Amygdaloideae | Contoneaster sp.,Melburne, Australia, 1998 |
| LA058 | Amygdaloideae | Oregon state university, OR, USA |
| LA070 | Amygdaloideae | " |
| LA075 | Amygdaloideae | " |
| LA082 | Amygdaloideae | " |
| LA 100 | Amygdaloideae | " |
| LA511 | Amygdaloideae | " |
| LA515 | Amygdaloideae | " |
| LA522 | Amygdaloideae | " |
| LA536 | Amygdaloideae | " |
| LA635 | Amygdaloideae | " |
| LA636 | Amygdaloideae | " |
| LA637 | Amygdaloideae | " |

**EXAMPLE 2** - **Materials and methods**

**Isolation and purification of bacteriophages**

Phages were isolated from apple orchards, which was collected in Romania, Hungary and Spain. Phage was isolated according method which is provided by Gill et.al [Gill, J. J., Svircev, A. M., Smith, R., & Castle, A. J. (2003). Bacteriophages of Erwinia amylovora. Applied and Environmental Microbiology, 69(4), 2133-2138.] Phage detection, Purification and identification of the phages were done with a combination of spot test, double agar overlay and qPCR methods [Adams, M.H. (1959). Bacteriophages. New York: Inter Science Publishers. 27. Kutter E. Phage host range and efficiency of plating. Methods Mol Biol. 2009;501:141-9. Kropinski AM, Mazzocco A, Waddell TE, Lingohr E, Johnson RP. Enumeration of bacteriophages by double agar overlay plaque assay. Methods Mol Biol. 2009;501:69-76. Gayder S, Parcey M, Castle AJ, Svircev AM. Host Range of Bacteriophages Against a World-Wide Collection of Erwinia amylovora Determined Using a Quantitative PCR Assay. Viruses. 2019 Oct 1;11(10):910.]

**Plaque morphology, quantitative and qualitative evaluation of bacteriophage**

To evaluate the infectivity of phage and morphology as well as performed quantitative, double agar overlay method[Kropinski A. M. et al., 2009], and qualitative spot test [Kutter E. Phage host range and efficiency of plating. Methods Mol Biol. 2009;501:141-9.], examinations. Based on the double agar method was characterized plaque morphology of the phage. Infectivity was represented as the efficiency of plating (EOP) [Kropinski AM, Mazzocco A, Waddell TE, Lingohr E, Johnson RP. Enumeration of bacteriophages by double agar overlay plaque assay. Methods Mol Biol. 2009;501:69-76. Duarte J, and Pereira C, Bacteriophages with Potential to Inactivate Aeromonas hydrophila in Cockles: In Vitro and In Vivo Preliminary Studies. Antibiotics (Basel). 2021 Jun 12;10(6):710.]. EOP was calculated:

$$\mathrm{EOP} = \frac{T}{T_0} \quad (1)$$

where:
T- Titer of phage with tested bacteria, $T_0$ - Titer of phage with host bacteria.
If E=1 Tested bacteria has the same output of phage as the host strain, E> 1 Tested bacteria Should be considered a better host strain, E< Tested bacteria has less output of phage as host strain.
Spot test results were analyzed according to Kutter E., 4+ - confluent lysis (CL) 3+ - semi-Confluent lysis (SCL), 2+ Confluent lysis with opacity duo to secondary growth (OL), 1+ - on the bacterial growth, individual lysis sites are represented as small dots (IPO) and no lysis [Kutter E. 2009, see above]

**_In vitro_ infection assay**

To determine the efficiency of phages and cocktail in a liquid medium with Hungarian _Erwinia amylovora_ strains was used multi-plate reader assay. Bacterial night cultures were adjusted to OD= 0.50, then was diluted 20 X in 1 ml of LB broth. In each well of 96 well-plate was added: 160 $\mu$l LB, 20 $\mu$l bacterial suspension, and 20 phages from sufficient dilution to get MOI = 0.1, incubation at 26°C, for 24 hours, with medium shaking, OD 600nm was recorded 30 minutes. For each bacterium was done in 3 replicates.

**Stability of bacteriophage**

The stability of the phage was tested at different temperatures [Kim, S.G.; Jun, J.W.; Giri, S.S.; Yun, S.; Kim, H.J.; Kim, S.W.; Kang, J.W.; Han, S.J.; Jeong, D.; Park, S.C. Isolation and characterisation of pVa-21, a giant bacteriophage with anti-biofilm potential against Vibrio alginolyticus. Sci. Rep. 2019, 9, 6284.]. Phage suspension was incubated at temperatures 50°C, 60°C and 70°C for 30 sec, 60 sec, and 120 sec. Numbers of phages in various time points were recorded and compared to the full
From each suspension was aliquoted 100 $\mu$l and diluted with serial dilutions. Phages titer was evaluated by spot test [Adams, M.H. (1959). Bacteriophages. New York: Inter Science Publishers. Kutter E. Phage host range and efficiency of plating. Methods Mol Biol. 2009;501:141-9.].
The desiccation tolerance of the phage was also examined. As in the open area desiccation process is common, on 3 glass slides dripped 10 $\mu$l of phage. Sampling was done for the following periods: T=0, T=30 minutes, and T= 24 hours. After drying, for sample $T_0$ was added 30 $\mu$l SM buffer and mixed well with pipette tip, 20 $\mu$l was diluted in 180 $\mu$l, then done other 10x dilutions from $10^{-2}$ to $10^{-9}$. The same way was tested slide samples after 30 minutes and 24 hours incubation. Phages titer was evaluated by spot test [Adams, M.H. (1959).; Kutter E. (2009), see above].
Since in the open environment plants are under sunlight exposition which can affect the viability of the phages [Born Y, Bosshard L, Duffy B, Loessner MJ, Fieseler L. Protection of Erwinia amylovora bacteriophage Y2 from UV-induced damage by natural compounds. Bacteriophage. 2015 Jul 24;5(4):e1074330. Jones JB, Vallad GE, Iriarte FB, Obradović A, Wernsing MH, Jackson LE, Balogh B, Hong JC, Momol MT. Considerations for using bacteriophages for plant disease control. Bacteriophage. 2012 Oct 1;2(4):208-214.], Cocktail phages were tested on UV tolerance with UV-B similarly as described in Kaiser et al., 2018 [Kaiser D, Bacher S, Mène-Saffrané L, Grabenweger G. Efficiency of natural substances to protect Beauveria bassiana conidia from UV radiation. Pest Manag Sci. 2019 Feb;75(2):556-563.]. The phages were tested with and without UV protectant PABA and surfactant Atlox AL-2575. Phages titer was evaluated by spot test [Adams, M.H. (1959). 27.Kutter E. (2009), see above].

**Liquid propagation**

Phage liquid propagation was conducted in the following way: into an Erlenmeyer flask (plugged with cotton-wool paper) with 57 ml of LB broth + 3 ml of 1M MgCh was added 1 ml bacterial culture and incubated at $26^0$C for 3 hours with 160 rpm shaking. After incubation was measured OD of bacterial suspension. The concentration of bacteria was calculated based on the standard curve. Considering the concentration of the bacterial suspension was calculated volume and concentration of phages were added to have MOI=1, MOI=0.1, and MOI=0.01. After adding phages flasks were incubated at 26°C overnight, with 160 rpm shaking. After overnight incubation, liquid suspension was transferred into 50 ml centrifuge tubes. centrifuged at 3500 g for 25 minutes, then the supernatant was filtered via 0.22 $\mu$m filters. Stored at 4 °C. titer was determined via double agar overlay method [Adams, M.H. (1959). Kropinski A.M. (2009), see above].

**DNA Isolation and sequence**

From purified phage, the suspension was isolated DNA via a High Pure viral Nucleic acid kit (Roche Diagnostics GmbH, Germany), according to manufacturer instructions. For sequence, the DNA library was prepared with a Nextera XT DNA Library Prep Kit (Illumina Inc., USA)
according to manufacturer instructions. Phage DNA was sequenced by an Illumina MiSeq System (Illumina Inc., Seoul, USA). Assemble of sequence data performed using SPAdes assembler. ORFs were predicted and the genome was annotated by the Rast server (https://rast.nmpdr.org ), the genome was blasted by Nucleoid Blast (https:/iblast.ncbi.nlm.nih.gov/Blast.cgi).

**Primer design and qPCR host range**

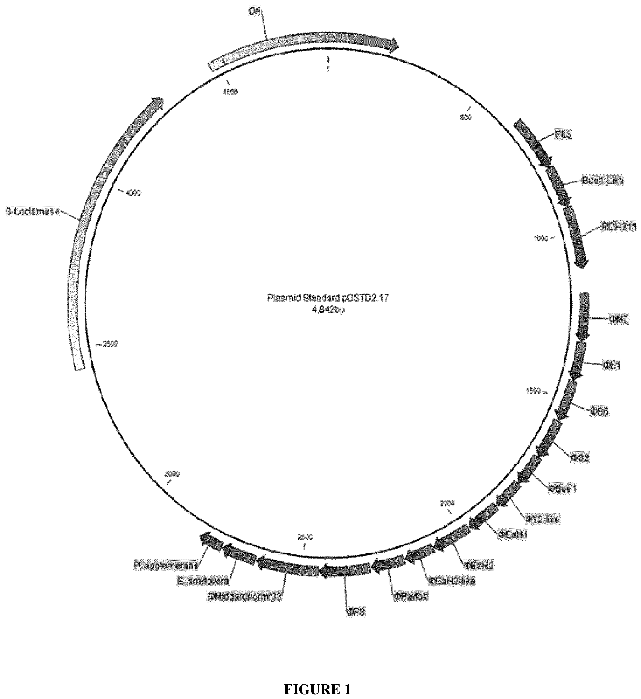

For performing quantitative PCR was designed primers, probes and plasmid standard pQSTD2.17 which contains the amplicons for the 15 phage strains, *Erwinia amylovora,* and *Pantoea agglomerans* (Figure 1). Procedures were conducted according to Gayder et al., in particular, the phage host range was determined by the qPCR method [Gayder S, Parcey M, Castle AJ, Svircev AM. Host Range of Bacteriophages Against a World-Wide Collection of Erwinia amylovora Determined Using a Quantitative PCR Assay. Viruses. 2019 Oct 1;11(10):910.].

Bacterial night culture was prepared in a 96-well plate with 200 $\mu$l LB, grown for 24 h at $28^0$C with 600 rpm. After overnight incubation bacteria were re-incubated in 160 $\mu$l LB has added 3 ml of the night culture. After cells were diluted to get approximately $1 \times 10^6$ genomes/mL of the host. In each well was aliquoted 190 $\mu$l of the phage's lysate with titer $10^6$, and 10 $\mu$l of diluted bacterial suspension was added and incubated for 8 h at $28^0$C with 600 rpm. The mixture was heated at $80^0$C for 30 minutes to heat-kill bacteria and stop the experiment. the phage genome was qualified using quantitative PCR, without DNA extraction. All bacteria were tested with phage in 3 replicates. Figure.1 shows the map of the plasmid pQSTD2.17 used in quantitative PCR reactions.

**Quantitative qPCR**

For the purification process and quantitative evaluation of phages use qPCR method, for qPCR was used GoTaq Probe qPCR kit (Promega Corporation, USA). Each reaction contained 2 $\mu$l of the sample, 200 nM of each primer and probe in a total 20 $\mu$l reaction mix. The PCR reaction was performed in the following steps: 10 minutes preheating at 95°C, then 40 cycles of 15 sec at 95°C, 15 sec at 55 $^0$C, and collecting data for 30 sec at $60^0$C. For quantitative evaluation of phages, Plasmid Standard pQSTD2.17. has been used [Parcey M, Gayder S, Castle AJ, Svircev AM. Molecular Profile of Phage Infection: A Novel Approach for the Characterization of Erwinia Phages through qPCR. Int J Mol Sci. 2020 Jan 15;21(2):553.]

**EXAMPLE 3 - Bacteriophage and its use against *Erwinia amylovora***

**Results**

From the apple samples of the phage suspension, phage lysis activity was detected via Spot test. After purification from phage suspension, received 1-1.5 mm size plaques, it took 3 stages with a double-agar method to have mono-morphological plaques. In Paraller with double agar method, after every stage of purification phage plaques were tested by qPCR with already existing phage's primers [Gayder, S.; Parcey, M.; Nesbitt, D.; Castle, A.J.; Svircev, A.M. Population Dynamics between Erwinia amylovora, Pantoea agglomerans and Bacteriophages: Exploiting Synergy and Competition to Improve Phage Cocktail Efficacy. Microorganisms 2020, 8, 1449.; Born Y, Fieseler L, Marazzi J, Lurz R, Duffy B, Loessner MJ. Novel virulent and broad-host-range Erwinia amylovora bacteriophages reveal a high degree of mosaicism and a relationship to Enterobacteriaceae phages. Appl Environ Microbiol. 2011 Sep;77(17):5945-54.; Dömötör, D., Becságh, P., Rákhely, G., Schneider, G., & Kovács,T. (2012). Complete genomic sequence of Erwinia amylovora phage PhiEaH2. Journal of Virology, 86(19), 10899-10899.; Meczker, K., Dömötör, D., Vass, J., Rákhely, G., Schneider, G., & Kovács, T. (2014). The genome of the Erwinia amylovora phage PhiEaH1 reveals greater diversity and broadens the applicability of phages for the treatment of fire blight. FEMS Microbiology Letters, 350(1), 25-27.].

Results are given in Table 2b. Samples were negative with the given phage's primers.

Table 2b. Results of qPCR checking of Ea PF8 Y2 corresponds to PF1)

| Primers of phage | Y2 | M7 | L1 | S6 | S2 | Bue1 | EaH1 | EaH2 | 37-70 |
|---|---|---|---|---|---|---|---|---|---|
| Ea PF8 | - | - | - | - | - | - | - | - | - |

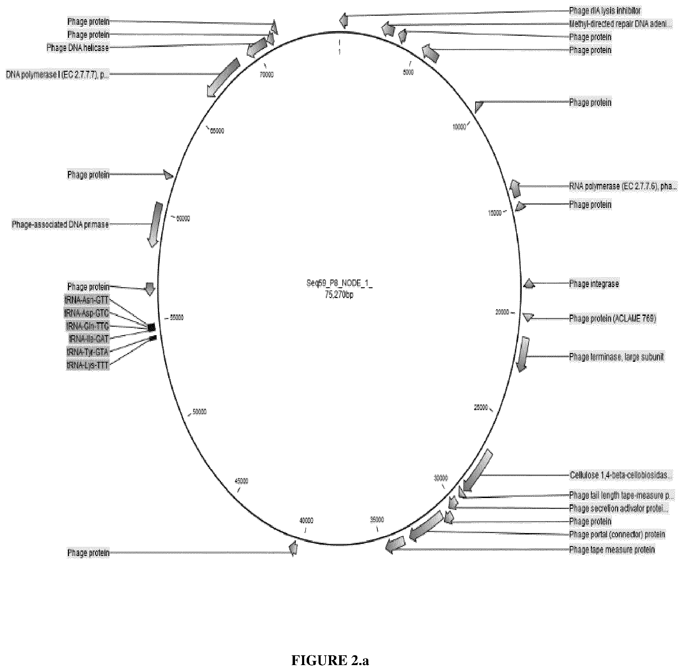

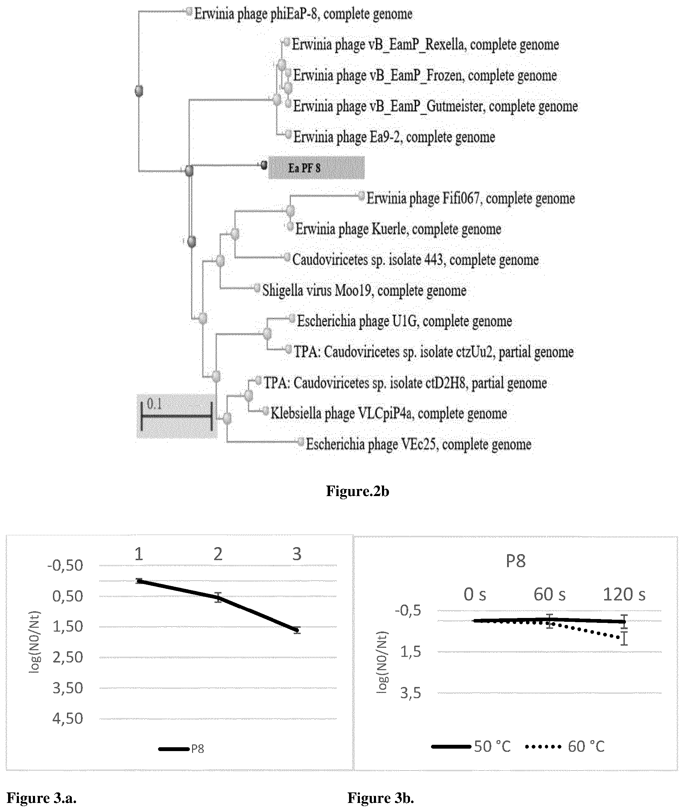

In the result of sequencing and assembling of phage, phage Ea PF8 was detected to have 75,270 bps DNA with 48.1% content of a G-C. In phage, using the RAST server was predicted 89 open reading frames (ORF). The function of most ORFs is not detected, so they are defined as hypothetical proteins, but there are also some functional ones, total data are given on the Genome map of the phage sequence in Figure 2. Genome blast results show, that most phage is related with 78.1 % identity to the phage phiEap-8 [40], the full tree is presented in Figure 2b. In order to perform evaluation of phage effectiveness and host range with quantitative PCR, were designed primers and probe Table 3.

Figure 2a. shows Genome map of the phage Ea PF 8.

Figure.2b shows the phylogenetic tree of the phage Ea PF8.

Table 3. Ea PF 8 phage primers and probe characteristics, F-Forward primer, R- Reverse primer, P-probe.

| Name | | Oligo Sequence | Length | Amplicon Length | Amplicon |
|---|---|---|---|---|---|
| P8-F | SEQ ID NO: 17 | GTGGATCCAC TCAGTGCACA | 20 | 154 SEQ ID NO: 20 | GTGGATCCACTCAGTGCACAGCTCTC GCAAGCCATGCAGCCGAACTCGACAG ATCTCTTGAACAAGGCCGATCTTTGG TACGAGAACTCAGGGAAACTCTTAGA TTCCGTGACGAGCAACTCCGGGCAGT AAGTGCTAAATTGCTCGCTGACCG |
| P8-R | SEQ ID NO: 18 | CGGTCAGCGA GCAATTTAGC | 20 | | |
| P8-P | SEQ ID NO: 19 | CCGTGACGAG CAACTCCGGG | 20 | | |

*Comparison of Ea PF 8 sequence with other prior art phage sequences*

Apparently one of the closest prior art solutions are disclosed in KR102025403B1. While the sequence of the deposited bacteriophage effective against *Erwinia amylovora* is not disclosed in KR102025403B1, sequence alignment results are available with publicly available accession numbers of 6 different types of phage genomes, specifically

Erwinia amylovora phage phiEa104 complete genome, (FQ482083.1)
Erwinia phage phiEa21-4, complete genome, (EU710883.1)
Erwinia phage vB_EamM-M7. complete genome, (HQ728263.1)
Erwinia phage vB.EamP.Frozen. complete genome, (KX098389.2)
Erwinia phage v8_EamP_Gutmeister, complete genome, (KX098391.1)
Erwinia phage Ea9-2, complete genome (KF806588.1)

which were showed to have a high similarity with the strain of KR102025403B1 (figure 9). Based on the presented table, local and global sequence alignment comparisons were performed between the new P8 sequence and existing genomes.

We have used the downloadable desktop version of BLAST (Basic Local Alignment Search Tool) (version blastn 2.15.0+) for sequence comparision of PF 8 phage sequence and six different genomes: FQ482083.1, EU710883.1, HQ728263.1, KX098389.2, KX098391.1, KF806588.1. and the command line tool of EMBOSS' Needle (version EMBOSS:6.6.0.0) was utilized for global Needleman-Wunsch algorithm based alignment.

Accession numbers from group 7 (FQ482083.1, EU710883.1, HQ728263.1) all provided a 47.7% identity / similarity score, while accessions from group 8 (KX098389.2, KX098391.1, KF806588.1) performed 48.4% identity score.

Thus, for similarity comparison the newly claimed P8 phage genome sequence share < 50% sequence identity of above-mentioned prior art genome sequences.

*Characterization of Ea PF-8 Phage phage*

Ea PF-8 Phage susceptibility was evaluated against 37 *Erwinia amylovora* bacteria from the National Collection of Agricultural and Industrial Microorganisms (Hungary) (see Table 1), it is essential to evaluate both the qualitative and quantitative effectiveness of phages Table 4. The inventors performed 4 different methods: i) spot test was done to determine lysis activity, ii) double agar method for EOP calculation and iii) qPCR method was used for quantitative evaluation, whereas iv) the 96-well plate method to determine the efficiency of phage in liquid with an uninterrupted time regime. With spot test it was shown that phage is available to lyse all bacteria but with different quality: confluent lysis (4+) was present in 41% of the bacteria, semi-confluent lysis (3+) was present in 43% of the bacteria, opaque lysis was (2+) in 16 % of them. The efficiency of planting plating was more than 0.7 in 81% of Ea strains compared with a host. Efficacy of the Ea PF8 phage determined by 96-well multi-plate reader runs was as follows: more than 80% - in 65 % of the tested Ea bacteria, from 50 - 70 % - in 10 % of the tested Ea bacteria, from 0-50 % - in 25 % of the tested Ea bacteria. QPCR showed that more phages were able to produce at least $10^{11}$ genome copies/ml - in the case of 70% of the tested bacteria.

The effectiveness of the phage Ea PF 8 was determined with worldwide collection of the *Erwinia amylovora* and *Pantoea agglomerance* strains by quantitative PCR, a total 108 strains. As results show Ea pF 8 phage was effective and can produce more than $10^9$ genome copies/ml in 67% of tested bacteria strains.

Table 4. Quantitative and Qualitative evaluation of phage effectivity with Hungarian *Erwina amylovora* Strains

| *Erwinia Amyvolora* strains | EOP | Lysis activity | Genome copies/ml | Efficacy % |
|---|---|---|---|---|
| Ea B 01272 | 0.7 | 3 | 32721403712 | 75 % |
| Ea B 01616 | 0 | 3 | 15936863117 | 50 % |
| Ea B 01728 | 0.9 | 4 | 2.62584E+11 | 80 % |
| Ea B 01729 | 0.9 | 4 | 1.39058E+11 | 85 % |
| Ea B 01731 | 0.8 | 3 | 2.20317E+11 | 30 % |
| Ea B 01733 | 0.5 | 2 | 6998429121 | 100 % |
| Ea B 01734 | 0.4 | 3 | 2.61703E+11 | 95 % |
| Ea B 01735 | 0.6 | 4 | 2.09325E+11 | 60 % |
| Ea B 01738 | 0.7 | 3 | 6591862336 | 80 % |
| Ea B 01756 | 0.7 | 3 | 58918932.57 | 100 % |
| Ea B 01757 | 0.3 | 2 | 1.69E+11 | 85 % |
| Ea B 01840 | 0.8 | 3 | 3.34154E+11 | 70 % |
| Ea B 01843 | 1 | 4 | 1.35877E+11 | 100 % |
| Ea B 01844 | 1.1 | 4 | 2.01402E+11 | 80 % |
| Ea B 01853 | 0.27 | 2 | 2175641926 | 50 % |
| Ea B 01855 | 0.09 | 2 | 1270344127 | 20 % |
| Ea B 01896 | 0.5 | 2 | 35364680136 | 30 % |
| Ea B 01898 | 0.8 | 3 | 179004.0588 | 40 % |
| Ea B 01901 | 0.8 | 3 | 19638905614 | 70 % |

(continued)

| Erwinia Amyvolora strains | EOP | Lysis activity | Genome copies/ml | Efficacy % |
|---|---|---|---|---|
| Ea B 01903 | 0.75 | 4 | 3.01402E+11 | 70 % |
| Ea B 01905 | 0.8 | 3 | 1.13842E+11 | 50 % |
| Ea B 01906 | 0.7 | 3 | 31569582431 | 55 % |
| Ea B 01960 | 0.5 | 2 | 33290152.03 | 80 % |
| Ea B 01961 | 0.6 | 3 | 3266584433 | 40 % |
| Ea B 01962 | 0.7 | 3 | 12256931583 | 0 % |
| Ea B 01963 | 0.5 | 3 | 31664882471 | 10 % |
| Ea B 01964 | 0.7 | 3 | 26639041590 | 80 % |
| Ea B 01971 | 1 | 4 | 2.26351E+11 | 85 % |
| Ea B 01972 | 0.96 | 4 | 1.2639E+11 | 85 % |
| Ea B 01973 | 0.96 | 3 | 2.56325E+11 | 80 % |
| Ea B 01975 | 1.2 | 4 | 2.35611E+11 | 95 % |
| Ea B 01978 | 1 | 4 | 2.61703E+11 | 100 % |
| Ea B 01980 | 0.8 | 4 | 67538601788 | 90 % |
| Ea B 01983 | 0.5 | 4 | 2.80334E+11 | 100 % |
| Ea G254 | 0.7 | 4 | 2.06548E+11 | 85 % |
| Ea G255 | 0.85 | 4 | 2.98521E+11 | 85 % |
| Ea B 0118T | 0.7 | 4 | 1.66012E+11 | 80 % |

| Strain | Group | Origin of the Stain | Genome copies/ml |
|---|---|---|---|
| CFBP 1430 | Amygdaloideae | CIRM-CFBP French Collection for Plant Associated Bacteria | 6743333333 |
| 4/82 | Amygdaloideae | P. communis, Egypt, 1982, W. Zeller | 2.0433E+10 |
| Ea 56400 | Amygdaloideae | Pyrus sp., Fribourg CH, 2007 | 3673333333 |
| 01SFR-BO | Amygdaloideae | Italy, 1991 [41] | 3.7767E+10 |
| LA477 | Amygdaloideae | Oregon state university, OR, USA | 2448333333 |
| LA071 | Amygdaloideae | " | 1.2213E+10 |
| Ea644 | Amygdaloideae | USA [41] | 6365333333 |
| Tk119 | Amygdaloideae | Pyrus sp, Izmir, Turkey, 2011 | 3803333333 |
| Ea263 | Amygdaloideae | Cydonia so, Israel, 1997. | 2.9033E+10 |
| UPN527 | Amygdaloideae | Spain,1996 [41] | 6.03E+10 |
| LebA3 | Amygdaloideae | Malus sp.,Lebanon,1998 | 1.8333E+10 |
| SAE3 | Amygdaloideae | Malus sp., Saratov, Russia, 2012 | 2273666667 |
| MOE1 | Amygdaloideae | Malus sp., Moldova, 2007 | 2430333333 |
| TE4 | Amygdaloideae | Malus sp., Tambov, Russia, 2007 | 1482000000 |
| Tk86 | Amygdaloideae | Cydonia sp., Bursa, Turkey,2007 | 2256000000 |
| KBE1 | Amygdaloideae | Cydonia sp., Kabardino-Balkaria, Russia, 2009 | 3786666667 |
| VGE1 | Amygdaloideae | Cydonia sp., Volgograd, Russia, 2010 | 828666667 |
| Ea2 | Amygdaloideae | Pyrus sp., Lorestan, Iran, 2009 | 3.1933E+10 |
| CFBP 1349 | Amygdaloideae | CIRM-CFBP French Collection for Plant Associated Bacteria | 2602666667 |

(continued)

| Strain | Group | Origin of the Stain | Genome copies/ml |
|---|---|---|---|
| Ea33 | Amygdaloideae | Cydonia sp., Semnan, Iran,2009 | 4046666667 |
| Ea1 | Amygdaloideae | Pyrus sp., Iran, 2009 | 4963333333 |
| VRE4 | Amygdaloideae | Malus sp., Voronezh, Russia, 2010 | 2431333333 |
| CFBP3098 | Amygdaloideae | CIRM-CFBP French Collection for Plant Associated Bacteria | 8693333.33 |
| CFBP3860 | Amygdaloideae | " | 3.3633E+10 |
| OR29 | Amygdaloideae | Hood River, Oregon state university, OR, USA | 2700000000 |
| LebB66 | Amygdaloideae | Cydonia sp., Lebanon, 1998 | 1.6527E+10 |
| UTRJ2 | Amygdaloideae | Malus sp,. UT, USA, 2000 | 2430000 |
| CFBP3792 | Amygdaloideae | CIRM-CFBP French Collection for Plant Associated Bacteria | 45300000 |
| TK159 | Amygdaloideae | Cydonia sp., Zonguldak, Turkey, 2012 | 2455666667 |
| 87-73 | Amygdaloideae | Malus sp., WA,Usa, | 2856000000 |
| BH | Amygdaloideae | Malus sp., MI, USA, 2008 | 2932000000 |
| CA3R | Amygdaloideae | Malus sp., Ca, USA, 1995 | 870266667 |

**Table 5** Quantative PCR evaluation of the EA PF 8 phage effectivenes with *Erwinia amylovora* strains.

| | | | |
|---|---|---|---|
| FB93-9 | Amygdaloideae | Malus sp., ID, USA | 26893333.3 |
| NW1-1 | Amygdaloideae | Malus sp., MI, USA, 2011 | 75953333.3 |
| OKR1 | Amygdaloideae | Rubus sp., OK, USA, 2002 | 5273333333 |
| RBA4 | Amygdaloideae | Rubus sp., MI, USA | 227000000 |
| RKK3 | Amygdaloideae | Rubus sp., MI, USA | 1.1097E+10 |
| TxLo3 | Amygdaloideae | Loquat, TX, Usa,2011 | 711666667 |
| WSDA16 | Amygdaloideae | Washington State Department of Agriculture, USA | 2704333333 |
| SE1 | Amygdaloideae | Malus sp., Samara, Russia, 2008 | 3006666667 |
| SE11 | Amygdaloideae | Malus sp., Samara, Russia, 2012 | 1707666667 |
| FEa9 | Amygdaloideae | Pyracantha sp.,Wojewodztwo Mazowiecki, Poland,2011 | 1195000000 |
| KE9 | Amygdaloideae | Crataegus sp.,Kaliningrad, Russia, 2003 | 4426666667 |
| Ea-32 | Amygdaloideae | Cydonia sp., East-Azerbaizjan, Iran, 2009 | 1.565E+10 |
| Ea-275 | Amygdaloideae | Cotoneaster sp., Stammheim, CH,1989 | 5480000000 |
| CFBP3049 | Amygdaloideae | CIRM-CFBP French Collection for Plant Associated Bacteria | 3355666667 |
| Ea646 | Amygdaloideae | Rubus sp., USA | 263333333 |
| Ea625a | Amygdaloideae | Rubis so., USA, 1996 | 177733333 |
| Ea530 | Amygdaloideae | Rubus sp., USA, 1949 | 191433333 |
| Ea592 | Amygdaloideae | Rubus sp., USA, 1995 | 51066666.7 |
| 393-3NZ | Amygdaloideae | Malus sp., Nelson, South Island, New Zealand, 1998 | 3336666667 |
| 8665NZ | Amygdaloideae | Malus sp., Appleby, South Island, New Zealand, 1984 | 4060000000 |
| JL1185 | Amygdaloideae | Pyrus sp., Quincy, WA, USA | 361333333 |
| CFBP1232 | Amygdaloideae | CIRM-CFBP French Collection for Plant Associated Bacteria | 5656666667 |
| CFBP2586 | Amygdaloideae | " | 2823333333 |
| JL1189 | Amygdaloideae | Pyrus sp., Granger, WA, USA, 1988 | 5.3933E+10 |

(continued)

| | | | |
|---|---|---|---|
| IL-5 | Amygdaloideae | Rubus sp., USA, 1977 | 1305800000 |
| IH3-1 | Amygdaloideae | Indian Hawthorn, LA, USA, 1998 | 136333333 |
| OR25 | Amygdaloideae | Hood River, Oregon state university, OR, USA | 1705000000 |
| UTFer2 | Amygdaloideae | Malus sp., UT, USA, 2000 | 3683333.33 |
| LA025 | Amygdaloideae | Oregon state university, OR, USA, 1988 | 2236666667 |
| LA036 | Amygdaloideae | " | 2376666.67 |
| LA096 | Amygdaloideae | " | 1713333333 |
| LA 102 | Amygdaloideae | " | 1013000000 |
| JL1168 | Amygdaloideae | Pyrus sp., Quincy, WA, USA, 1988 | 3.9067E+10 |
| JL1170 | Amygdaloideae | Pyrus sp., Quincy, WA, USA, 1988 | 377666667 |
| LA092 | Amygdaloideae | Oregon state university, OR, USA | 4950000000 |
| AFRS2 | Amygdaloideae | Malus sp., Alabama, USA, 1994 | 1293666667 |
| MO-35 | Amygdaloideae | Moldavia, Malus sp., 2007 | 258700000 |
| MO-E-101b | Amygdaloideae | Moldavia, Malus sp., 2007 | 2693333.33 |
| SLAPL#3 | Amygdaloideae | Malus sp., Ca, USA, 1995 | 28363333.3 |
| CFBP2301 | Amygdaloideae | CIRM-CFBP French Collection for Plant Associated Bacteria | 2530000000 |
| Ea209 | Amygdaloideae | Agricultural Reasearch oraganization of Israel, Bet-Degan, Israel | 3383333333 |
| MR-1 | Amygdaloideae | USA [41] | 66366666.7 |
| CFBP3020 | Amygdaloideae | CIRM-CFBP French Collection for Plant Associated Bacteria | 4220000000 |
| CFBP1252 | Amygdaloideae | " | 1.1953E+10 |
| Ea495 | Amygdaloideae | Sorbus sp., Ireland, 1986 | 2.69E+10 |
| Ea266 | Amygdaloideae | Malus sp., Ontario, Canada, 1983 | 47800000 |
| Ea01-03p | Amygdaloideae | Pyrus sp., Nova Scotia, Canada | 2223333.33 |
| PD2915 | Amygdaloideae | Amelanchier, Canada, 1996 | 16353333.3 |
| E7004M | Amygdaloideae | BC Canada | 6373333333 |
| IL-6 | Amygdaloideae | Rubus sp., USA 1998 | 1.244E+10 |
| CPFB1309 | Amygdaloideae | CIRM-CFBP French Collection for Plant Associated Bacteria | 2680000000 |
| 99.12 | Amygdaloideae | Cotoneaster sp., Auckland, Nort Island, New Zealand, 1999 | 2396666667 |
| 8862 | Amygdaloideae | Malus sp., Hastings, North Island, New Zealand, 1984 | 5540000000 |
| 13300 | Amygdaloideae | Contoneaster sp.,Melburne, Australia, 1998 | 3993333333 |
| LA058 | Amygdaloideae | Oregon state university, OR, USA | 2.8833E+10 |
| LA070 | Amygdaloideae | " | 3136666667 |
| LA075 | Amygdaloideae | " | 666166667 |
| LA082 | Amygdaloideae | " | 1.3157E+10 |
| LA100 | Amygdaloideae | " | 408133333 |
| LA511 | Amygdaloideae | " | 2422666.67 |
| LA515 | Amygdaloideae | " | 2.6633E+10 |
| LA522 | Amygdaloideae | " | 729333333 |
| LA536 | Amygdaloideae | " | 800333333 |
| LA635 | Amygdaloideae | " | 8400000000 |

(continued)

| LA636 | Amygdaloideae | " | 4686666667 |
|---|---|---|---|
| LA637 | Amygdaloideae | " | 4843333333 |

It was necessary to develop appropriate conditions for liquid propagation of the phages for further production. Based on the host range results *Erwinia Amylovora* UPN 527 was chosen as a host of the Ea PF 8. Liquid propagation was conducted in the 50 ml LB broth with a host bacterium, for liquid propagation was tested 3 different MOIs: 1, 0.1, and 0.01, the highest titer $10^{11}$ PFU/ml was received with MOI=0.1, and 0.01. One of the important characteristics of the phages is tolerance toward environmental stresses. Phage Ea PF 8 was tested at different temperatures and pH Figure 3. Phage seems to be stable after incubation at $50^0$C was the same, at 60°C after 120-sec phage PFU was decreased by 1 log. After 24 hours of desiccation a 1 log decrease of the phage PFU. Figure 3.a. shows desiccation tolerance, whereas 3.b temperature stability of Ea PF-8.

Phage Ea PF 8 was isolated from apple orchards from Hungary, as it was shown from DNA sequence analysis there is some relatedness to other phages, based on the percentage of this relatedness phage Ea PF 8 appears to be a new phage. The phage was tested with Hungarian as well as with other Erwinia amylovora strains from worldwide collections.

The results revealed that Ea PF 8 has a surprisingly broad host range, so we can consider it a lytic phage [Born Y, Fieseler L, Marazzi J, Lurz R, Duffy B, Loessner MJ. Novel virulent and broad-host-range Erwinia amylovora bacteriophages reveal a high degree of mosaicism and a relationship to Enterobacteriaceae phages. Appl Environ Microbiol. 2011 Sep;77(17):5945-54.], which is one of the important features of using phages against bacteria. So all results obtained during testing phage features such as genome sequence, morphology, effectiveness against target bacteria, and stability towards environmental stress is suggesting strong potential for its future use as an effective and realistic antibacterial agent against fire blight.

**EXAMPLE 4 -**

**Results**

From apple sample were detected phage lysis activity via Spot test. After was done purification from phage suspension, it took 3 stages with double-agar method to have mono-morphological plaques. In Paraller with double agar method, after every stage of purification phage plaques were tested by qPCR with already existing phage's primers. Results are in given in the Table 6.

**Table 6** Results of qPCR checking of the cocktail phages

| Primers of phage | Phi Y2 | Phi M7 | Phi L1 | Phi S6 | Phi S2 | Phi Bue1 | Phi EaH1 | Phi EaH2 |
|---|---|---|---|---|---|---|---|---|
| Ea PF 1 | Positive | Negative | Negative | Negative | Negative | Negative | Negative | Negative |
| Ea PF 2 | Negative | Negative | Positive | Negative | Negative | Negative | Negative | Negative |
| Ea PF 3 | Negative | Negative | Negative | Negative | Negative | Negative | Negative | Negative |
| Ea PF 8 | Negative | Negative | Negative | Negative | Negative | Negative | Negative | Negative |

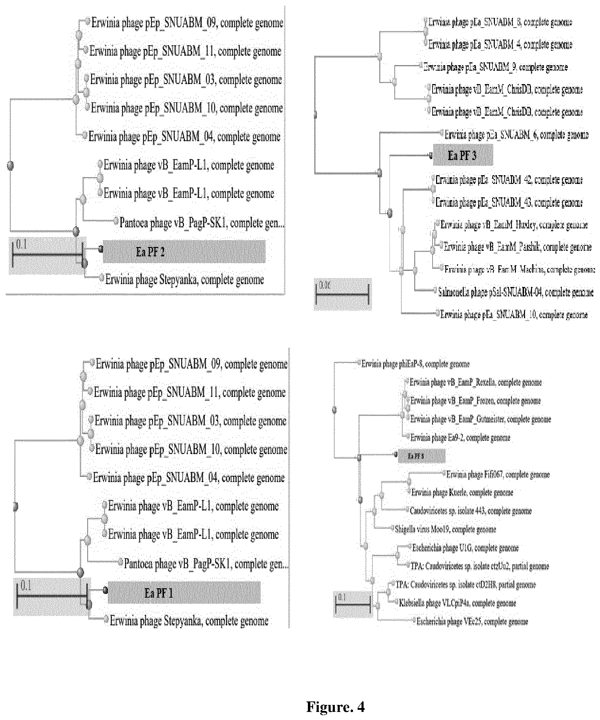

In the result of sequencing and assembling of phage Ea PF 1 was detected to have 53 802 bps DNA with a G-C which can be calculated from the sequence. In phage DNA there were detected 79 open reading frames (ORF)s. Function of most of ORFs is not detected, and were defined as hypotetical proteins, but we have also determined some functional ones, and total genome is presented as SEQ ID NO: 1. Genomic blast results show that phage is related with 92.98 % identity to SNAUB-27, full tree is presented as Figure 4. In order to perform phage effectiveness evaluation and host range, primers and probe were designed as well, to use more precision and accurate tools, see Table 7.

In the result of sequencing and assembling of phage Ea PF 2 was detected to have 39,879 bps DNA with 51.9 % content of a G-C. In phage DNA was detected 49 open reading frames (ORF)s. Function of half of ORFs is not detected, and were defined as hypotetical proteins, but we have also determined some functional ones, and total genome is presented as SEQ ID NO: 2. Genome blast results show, that phage is related with 92.98 % identity to Erwinia phage vB EamP-L1, full tree is presented as Figure 4. In order to perform phage effectiveness evaluation and host range, primers and probe were designed as well, to use more precision and accurate tools, see Table 7.

In the result of sequencing and assembling of phage Ea PF 3 was detected to have 242,924 bps DNA with 50.4% content of a G-C. In phage DNA was detected 283 open reading frames (ORF)s. Function of most of ORFs is not detected, and were defined as hypotetical proteins, but we have also determined some functional ones, and total genome is presented as SEQ ID NO: 3. Genome blast results show, that mostly phage is related with 88.88 % identity to Erwinia phage pEa_SNUABM_6, full tree is presented as Figure 4. In order to perform phage effectiveness evaluation and host range, primers and probe were designed as well, to use more precision and accurate tools, see Table 7.

In the result of sequencing and assembling of phage Ea PF 8 was detected to have 75,270 bps DNA with 48.1% content of a G-C. In phage DNA was detected 89 open reading frames (ORF)s. Function of most of ORFs is not detected, and were defined as hypotetical proteins, but we have also determined some functional ones, and total genome is presented as SEQ ID NO:4. Genome blast results showed that mostly phages are related with 78.1 % identity to Erwinia phage phiEaP8 , full tree is presented as Figure 4. In order to perform phage effectiveness evaluation and host range, primers and probe were designed as well, to use more precision and accurate tools, see Table 7. Figure 4 shows the phylogenetic trees of phages of the invention.

Table 7. Cocktail phages primers and probes characteristics, F-Forward primer, R- Reverse primer, P-probe.

| | Name | Length (bp) | | Full length |
|---|---|---|---|---|
| PF1 | 5-YP | 53 802 | SEQ ID NO: 1 | See the sequence listing |
| PF2 | C2 | 39 879 | SEQ ID NO: 2 | See the sequence listing |
| PF3 | PL3 | 242924 | SEQ ID NO: 3 | See the sequence listing |
| PF8 | P8 | 75 270 | SEQ ID NO: 4 | See the sequence listing |
| | | | | Oligo Sequence |
| PF-1 | Y2-like Forward primer | 22 | SEQ ID NO: 5 | AGTCTTTGTTGCCAGTGAGTGC |
| PF-1 | Y2-like Reverse primer | 20 | SEQ ID NO: 6 | ATCCGAAAGATGGCCGTAAC |
| PF-1 | Y2-like Probe | 22 | SEQ ID NO: 7 | CCGCCACCATACTGTACTGCCA |
| PF-1 | Y2-like Amplicon | 90 | SEQ ID NO: 8 | AGTCTTTGTTGCCAGTGAGTGCTTTCTTA CCGCCACCATACTGTACTGCCATGTATGG AGTCTTGATGAAGTTACGGCCATCTTTCG GAT |
| PF2 | L1- forward primer | 20 | SEQ ID NO: 9 | TTCGTCAAGGGATGATGCTG |
| PF2 | L1 - Reverse primer | 20 | SEQ ID NO: 10 | ATCTTGCGGATTCCATCACC |
| PF2 | L1 - Probe | 25 | SEQ ID NO: 11 | CGCTGAGTTCCTTGAGGCACATATG |
| PF2 | L1 Amplicon | 112 | SEQ ID NO: 12 | TTCGTCAAGGGATGATGCTGGATAGCGCA CAAGGCGAATCAGAGATGGTCGCTGAGTT CCTTGAGGCACATATGGAGCGTGAGGTGA TGGGCGGTGATGGAATCCGCAAGAT |
| PF3 | PL3 Forward primer | 20 | SEQ ID NO: 13 | ACCACGACATGATCCGTCAG |
| PF3 | PL3 Reverse primer | 20 | SEQ ID NO: 14 | GTGTCTGGGAACGGACAACA |
| PF3 | PL3 Probe | 20 | SEQ ID NO: 15 | CGGTCGGGAACGTTGTTTGC |
| PF3 | PL3 Amplicon | 162 | SEQ ID NO: 16 | ACCACGACATGATCCGTCAGCTGATTCGG TGTGTAGTTAAACGTCGAACCTGAACCGG GTAAATCGGTCGGGAACGTTGTTTGCCAG ACACGCAGGTGCTTCTTCTGGTTAAGCGT TCCGTCAAACATGACGGTGTAGTATTTGT TGTCCGTTCCCAGACAC |
| PF8 | P8 Forward primer | 20 | SEQ ID NO: 17 | GTGGATCCACTCAGTGCACA |
| PF8 | P8 Reverse primer | 20 | SEQ ID NO:18 | CGGTCAGCGAGCAATTTAGC |

(continued)

| | | | | Oligo Sequence |
|---|---|---|---|---|
| PF8 | P8 Probe | 20 | SEQ ID NO:19 | CCGTGACGAGCAACTCCGGG |
| PF8 | P8 Amplicon | 154 | SEQ ID NO:20 | GTGGATCCACTCAGTGCACAGCTCTCGCA AGCCATGCAGCCGAACTCGACAGATCTCT TGAACAAGGCCGATCTTTGGTACGAGAAC TCAGGGAAACTCTTAGATTCCGTGACGAG CAACTCCGGGCAGTAAGTGCTAAATTGCT CGCTGACCG |

The phage's susceptibility was evaluated against 37 *Erwinia amylovora* bacteria from National Collection of Agricultural and Industrial Microorganisms (Hungary), it is essential to evaluate both qualitative and quantitative effectiveness of phages. Altogether 4 different methods were performed: i) spot test was done for determination of lysis activity, ii) double agar method and iii) qPCR methods were used for quantitative evaluation and iv) 96-well plate method to determine efficiency of phage in liquid with uninterrupted time regime.

With spot test it was shown that phage Ea PF 1 is available to lyse 97 % bacteria, but with different quality: confluent lysis (4+) was present in 78 % of the bacteria, semi-confluent lysis (3+) was present in 14% of the bacteria, opaque lysis was (2+) in 5 % of the bacteria. Efficiency of plating was more than 0.7 in 82% of Ea strains compared with host. The efficacy of the Ea PF1 phage determined by 96-well multi plate reader runs was more than 80% in 46 % of the tested Ea bacteria, from 50 - 70 % in 27% of the tested Ea bacteria and from 0-50 % in 27 % of the tested Ea bacteria. QPCR showed that the phage was able to produce $10^{11}$ genome copies/ml in the case of 73% of the tested bacteria.

With spot test it was shown that phage Ea PF 2 is available to lyse 70 % bacteria, but with different quality: confluent lysis (4+) was present in 30 % of the bacteria, semi-confluent lysis (3+) was present in 30% of the bacteria, opaque lysis could be observed (2+) in 20 % of the bacteria. Efficiency of plating was more than 0.7 in 49% of Ea strains compared with host. The efficacy of the Ea PF 2 phage determined by 96-well multi plate reader runs was more than 80% in 19 % of the tested Ea bacteria, from 50 - 70 % - in 16 % of the tested Ea bacteria, from 0-50 % in 65 % of the tested Ea bacteria. QPCR showed that the phage was able to produce $10^{11}$ genome copies/ml in the case of 16% of the tested bacteria.

With spot test it was shown that phage Ea PF 3 is available to lyse 78 % bacteria, but with different quality: confluent lysis (4+) was resulted in 8 % of the bacteria, semi-confluent lysis (3+) was present in 38% of the bacteria, and opaque lysis could be observed (2+) in 32 % of the bacteria. Efficiency of plating was more than 0.7 in 32% of Ea strains compared with host. The efficacy of the Ea PF 3 phage determined by 96-well multi plate reader runs was: from 50 - 70 % in 23 % of the tested Ea bacteria and from 0-50 % in 77 % of the tested Ea bacteria. QPCR showed that phage was able to produce $10^{11}$ genome copies/ml in the case of 16% of the tested bacteria.

With spot test it was shown that phage Ea PF 8 is available to lyse all bacteria (100%), but with different quality: confluent lysis (4+) was present in 41% of the bacteria, semi-confluent lysis (3+) was present in 43% of the bacteria, and opaque lysis could be observed (2+) in 16 % of the bacteria. Efficiency of plating was more than 0.7 in 81% of Ea strains compare with host. Efficacy of the Ea PF8 phage determined by 96-well multi plate reader runs was: more than 80% in 65 % of the tested Ea bacteria, from 50 - 70 % in 10 % of the tested Ea bacteria, from 0-50 % in 25 % of the tested Ea bacteria. QPCR showed that the phage was able to produce $10^{11}$ genome copies/ml in the case of 70% of the tested bacteria.

As it has been shown that phages in the cocktail provide different quantitative and qualitative properties, it was surprisingly found that in cocktail a nearly full efficiency has been reached in a very broad spectrum of strains. Efficacy of the phage cocktail was determined by 96-well multi plate reader runs. It was 100% in 76% of the tested Ea bacteria, and from 80 - 95% in the remaining 24 % of the tested Ea bacteria.

Thus, the cocktail according to the invention provide an unexpectedly wide protection against the Fire blight disease.

With worldwide collection of Ea strains cocktail phages also showed very good results (see Table 8). QPCR showed that phage Ea PF 1 was able to produce $10^{10}$ genome copies/ml - in the case of 82% of the tested bacteria, Ea PF 2 was able to produce $10^{10}$ genome copies/ml - in the case of 49% of the tested bacteria, Ea PF 3 was able to produce $10^{10}$ genome copies/ml - in the case of 30 % of the tested bacteria and Ea PF 8 was able to produce $10^{10}$ genome copies/ml - in the case of 60 % of the tested bacteria.

**Table 8** Shows results of the host range with EOP determination

| Erwinia amylovora | Ea PF 1 | Ea PF 2 | Ea PF 3 | Ea PF 8 |
|---|---|---|---|---|
| Ea B 01272 | 0.7 | 1.1 | 0 | 0.7 |
| Ea B 01616 | 0 | 0 | 0 | 0 |
| Ea B 01728 | 0.6 | 1 | 0.6 | 0.9 |
| Ea B 01729 | 0 | 1 | 0.4 | 0.9 |
| Ea B 01731 | 0.4 | 0 | 0.2 | 0.8 |
| Ea B 01733 | 1.1 | 1 | 0.5 | 0.5 |
| Ea B 01734 | 0.7 | 0.4 | 0 | 0.4 |
| Ea B 01735 | 1.583333 | 0.9 | 0.242424 | 0.593333 |
| Ea B 01738 | 0.85 | 0.96 | 0.5 | 0.7 |
| Ea B 01756 | 0.6 | 0.3 | 0.2 | 0.7 |
| Ea B 01757 | 0.8 | 0.5 | 0.5 | 0.3 |
| Ea B 01840 | 1.183333 | 0.78 | 1.35 | 0.773333 |
| Ea B 01843 | 0.9 | 1.25 | 0.5 | 1 |
| Ea B 01844 | 0.75 | 1 | 0.9 | 1.1 |
| Ea B 01853 | 1.25 | 1.58 | 1.090909 | 0.27 |
| Ea B 01855 | 1.266667 | 1.446667 | 1.121212 | 0.09 |
| Ea B 01896 | 1.3 | 0.4 | 0.9 | 0.5 |
| Ea B 01898 | 1 | 0.12 | 0.95 | 0.8 |
| Ea B 01901 | 1 | 0 | 0.6 | 0.8 |
| Ea B 01903 | 0.616667 | 1.24 | 1.30303 | 0.75 |
| Ea B 01905 | 1.3 | 0.2 | 1.2 | 0 |
| Ea B 01906 | 0.8 | 0.4 | 0.6 | 0.7 |
| Ea B 01960 | 1 | 0 | 0 | 0.5 |
| Ea B 01961 | 0.8 | 0.5 | 1.2 | 0.6 |
| Ea B 01962 | 0 | 0.3 | 0 | 0.7 |
| Ea B 01963 | 1.4 | 1.5 | 0.4 | 0.5 |
| Ea B 01964 | 0.983333 | 1.52 | 0.69697 | 0.7 |
| Ea B 01971 | 0.85 | 0 | 0 | 1 |
| Ea B 01972 | 0.85 | 1 | 0.7 | 0.96 |
| Ea B 01973 | 0.85 | 0.9 | 0 | 0.96 |
| Ea B 01975 | 1.25 | 0 | 0 | 1.2 |
| Ea B 01978 | 1.25 | 0 | 0 | 1 |
| Ea B 01980 | 0.8 | 1 | 0.7 | 0.8 |
| Ea B 01983 | 0.8 | 0.95 | 0.5 | 0.5 |
| Ea G254 | 0.96 | 0 | 0 | 0.7 |
| Ea G255 | 1.1 | 0 | 0 | 0.85 |
| Ea B 0118T | 1 | 0 | 0.6 | 0.7 |

**Table 9.** Results of the host range with lysis quality evaluation

| Erwinia amylovora strains | Ea PF1 | Ea PF 2 | Ea PF 3 | Ea PF 8 |
|---|---|---|---|---|
| Ea B 01272 | 4 | 4 | 0 | 3 |
| Ea B 01616 | 0 | 2 | 3 | 3 |
| Ea B 01728 | 4 | 3 | 2 | 4 |
| Ea B 01729 | 4 | 3 | 2 | 4 |
| Ea B 01731 | 4 | 0 | 3 | 3 |
| Ea B 01733 | 4 | 4 | 3 | 2 |
| Ea B 01734 | 2 | 2 | 0 | 3 |
| Ea B 01735 | 3 | 2 | 1 | 4 |
| Ea B 01738 | 4 | 4 | 3 | 3 |
| Ea B 01756 | 3 | 1 | 1 | 3 |
| Ea B 01757 | 4 | 2.5 | 2 | 2 |
| Ea B 01840 | 4 | 4 | 3 | 3 |
| Ea B 01843 | 4 | 4 | 1 | 4 |
| Ea B 01844 | 4 | 4 | 3 | 4 |
| Ea B 01853 | 3 | 3 | 3 | 2 |
| Ea B 01855 | 4 | 3 | 3 | 2 |
| Ea B 01896 | 4 | 4 | 3 | 2 |
| Ea B 01898 | 4 | 2 | 3 | 3 |
| Ea B 01901 | 4 | 0 | 2 | 3 |
| Ea B 01903 | 3 | 2 | 3 | 4 |
| Ea B 01905 | 3 | 1 | 3 | 3 |
| Ea B 01906 | 4 | 2 | 2.5 | 3 |
| Ea B 01960 | 4 | 0 | 2 | 2 |
| Ea B 01961 | 4 | 3 | 3 | 3 |
| Ea B 01962 | 2 | 3 | 0 | 3 |
| Ea B 01963 | 4 | 3 | 1 | 3 |
| Ea B 01964 | 4 | 3 | 1 | 3 |
| Ea B 01971 | 4 | 0 | 4 | 4 |
| Ea B 01972 | 4 | 2 | 3 | 4 |
| Ea B 01973 | 4 | 4 | 1 | 3 |
| Ea B 01975 | 4 | 0 | 0 | 4 |
| Ea B 01978 | 4 | 0 | 0 | 4 |
| Ea B 01980 | 4 | 1 | 4 | 4 |
| Ea B 01983 | 4 | 4 | 2 | 4 |
| Ea G254 | 4 | 0 | 2 | 4 |
| Ea G255 | 4 | 0 | 4 | 4 |
| Ea B 0118T | 4 | 0 | 2 | 4 |

**Table 10.** Results of the host range with qPCR, genome copies/ml

| Erwinia amylovora strains | Ea PF 1 | Ea PF 2 | Ea PF 3 | Ea PF8 |
|---|---|---|---|---|
| Ea B 01272 | 3682441112 | 2.95E+09 | 535365.1 | 3.27E+10 |
| Ea B 01616 | 8.63E+09 | 1.56E+10 | 1.70E+11 | 1.59E+10 |
| Ea B 01728 | 3.48735E+11 | 2.224E+09 | 740229205 | 2.63E+11 |
| Ea B 01729 | 2.38546E+11 | 1.721E+11 | 7263850 | 1.391E+11 |
| Ea B 01731 | 2.36831E+11 | 1.697E+10 | 4.693E+10 | 2.2E+11 |
| Ea B 01733 | 1.891E+11 | 2.69E+08 | 5.61E+09 | 7E+09 |
| Ea B 01734 | 2.11503E+11 | 3.385E+09 | 240352629 | 2.617E+11 |
| Ea B 01735 | 3.84928E+11 | 1.517E+09 | 2.876E+10 | 2.093E+11 |
| Ea B 01738 | 3.21807E+11 | 1.561E+11 | 2.778E+10 | 6.59E+09 |
| Ea B 01756 | 1.27911E+11 | 6228002 | 13786726 | 58918933 |
| Ea B 01757 | 7.005E+10 | 1.39E+10 | 18393502 | 1.69E+11 |
| Ea B 1840 | 1.45991E+11 | 1.116E+11 | 1.181E+10 | 3.342E+11 |
| Ea B 01843 | 32170229998 | 1.145E+11 | 2.181E+11 | 1.359E+11 |
| Ea B 01844 | 1.07843E+11 | 1.454E+11 | 1.825E+11 | 2.014E+11 |
| Ea B 01853 | 28834752925 | 1.997E+10 | 1627010.7 | 2.176E+09 |
| Ea B 01855 | 1.44832E+11 | 2.1E+09 | 1.823E+09 | 1.27E+09 |
| Ea B 01896 | 1.89224E+11 | 6.498E+10 | 1.239E+09 | 3.536E+10 |
| Ea B 01898 | 711157867.8 | 6641270.3 | 7.409E+10 | 179004.06 |
| Ea B 01901 | 1.6355E+11 | 32268911 | 5263850 | 1.964E+10 |
| Ea B 01903 | 1.85872E+11 | 6.39E+09 | 1.598E+11 | 3.014E+11 |
| Ea B 01905 | 1.20142E+11 | 588557041 | 2.515E+10 | 1.138E+11 |
| Ea B 01906 | 1.15869E+11 | 1.54E+09 | 3.435E+10 | 3.157E+10 |
| Ea B 01960 | 2.3566E+11 | 168659.3 | 5391006 | 33290152 |
| Ea B 01961 | 2.33256E+11 | 1.047E+10 | 1.409E+11 | 3.267E+09 |
| Ea B 01962 | 2.35445E+11 | 53100239 | 233584665 | 1.226E+10 |
| Ea B 01963 | 3.89769E+11 | 4.155E+10 | 4.496E+10 | 3.167E+10 |
| Ea B 01964 | 97262812803 | 2.305E+09 | 7.458E+09 | 2.664E+ 10 |
| Ea B 01971 | 36994419599 | 1627010.7 | 1.061E+10 | 2.264E+11 |
| Ea B 01972 | 2.67391E+11 | 2.305E+09 | 6.639E+09 | 1.264E+11 |
| Ea B 01973 | 2.36831E+11 | 4.875E+09 | 32796.72 | 2.563E+11 |
| Ea B 01975 | 1.47353E+11 | 1.014E+09 | 31728.73 | 2.36E+11 |
| Ea B 01978 | 99242762692 | 3020013 | 4038766 | 2.617E+11 |
| Ea B 01980 | 71278415040 | 5.426E+10 | 1.475E+11 | 6.754E+10 |
| Ea B 01983 | 1.18292E+11 | 1.031E+11 | 254231610 | 2.803E+11 |
| Ea G254 | 3.25682E+11 | 518800398 | 9187738.8 | 2.065E+11 |
| Ea G255 | 2.95473E+11 | 970743179 | 85562154 | 2.985E+11 |
| Ea B 118T | 1.9778E+11 | 6640823.4 | 6.28E+09 | 1.66E+11 |

**Table 11.** Results of efficacy test of cocktail compared to single phages

| Erwinia amylovora strain | Cocktail | Ea PF 1 | Ea PF 2 | EA PF 3 | Ea PF8 |
|---|---|---|---|---|---|
| Ea B 01272 | 80 | 70 | 60 | 10 | 75 |
| Ea B 01616 | 100 | 70 | 20 | 30 | 50 |
| Ea B 01728 | 100 | 90 | 70 | 70 | 80 |
| Ea B 01729 | 100 | 95 | 100 | 10 | 85 |
| Ea B 01731 | 100 | 50 | 10 | 30 | 30 |
| Ea B 01733 | 100 | 70 | 60 | 20 | 100 |
| Ea B 01734 | 95 | 95 | 50 | 0 | 95 |
| Ea B 01735 | 90 | 60 | 20 | 20 | 60 |
| Ea B 01738 | 100 | 85 | 75 | 70 | 80 |
| Ea B 01756 | 100 | 70 | 10 | 10 | 100 |
| Ea B 01757 | 90 | 70 | 20 | 30 | 85 |
| Ea B 01840 | 100 | 40 | 40 | 40 | 70 |
| Ea B 01843 | 100 | 60 | 100 | 60 | 100 |
| Ea B 01844 | 100 | 100 | 100 | 70 | 80 |
| Ea B 01853 | 100 | 40 | 90 | 30 | 50 |
| Ea B 01855 | 90 | 20 | 20 | 20 | 20 |
| Ea B 01896 | 95 | 90 | 90 | 10 | 30 |
| Ea B 01898 | 100 | 50 | 20 | 80 | 40 |
| Ea B 01901 | 100 | 80 | 0 | 30 | 70 |
| Ea B 01903 | 100 | 30 | 10 | 30 | 70 |
| Ea B 01905 | 100 | 60 | 20 | 60 | 50 |
| Ea B 01906 | 100 | 50 | 30 | 30 | 55 |
| Ea B 01960 | 80 | 80 | 0 | 0 | 80 |
| Ea B 01961 | 100 | 40 | 10 | 25 | 40 |
| Ea B 01962 | 80 | 60 | 0 | 50 | 0 |
| Ea B 01963 | 100 | 40 | 40 | 10 | 10 |
| Ea B 01964 | 100 | 50 | 40 | 10 | 80 |
| Ea B 01971 | 100 | 85 | 0 | 70 | 85 |
| Ea B 01972 | 100 | 90 | 50 | 40 | 85 |
| Ea B 01973 | 80 | 70 | 80 | 10 | 80 |
| Ea B 01975 | 100 | 95 | 0 | 0 | 95 |
| Ea B 01978 | 100 | 80 | 0 | 0 | 100 |
| Ea B 01980 | 100 | 80 | 10 | 40 | 90 |
| Ea B 01983 | 100 | 80 | 70 | 40 | 100 |
| Ea G254 | 100 | 85 | 0 | 20 | 85 |
| Ea G255 | 100 | 60 | 0 | 25 | 85 |
| Ea B 0118T | 90 | 95 | 20 | 0 | 80 |

It was necessary to develop appropriate conditions for liquid propagation of the phages for further production. Phages were propagated in the 50 ml LB broth with a host bacteria, for liquid propagation was tested 3 different MOI: 1, 0.1 and 0.01, with MOI=0.1 and 0.01

Table 12. Results of efficacy test of cocktail compared to single phages

| Ea strains | Phage | Concentration PFU/ml MOI =1 | Concentration PFU/ml MOI=0.1 | Concentration PFU/ml MOI =0.01 |
|---|---|---|---|---|
| Ea SFR 180 | Ea PF 1 | $2\ 10^9$ | $4\ 10^{11}$ | $3.4\ 10^{11}$ |
| Ea CFBP 1430 | Ea PF 2 | $3.5\ 10^9$ | $1.2\ 10^{11}$ | $2\ 10^{11}$ |
| Ea OR 25 | Ea PF 3 | $1.3\ 10^9$ | $2\ 10^{10}$ | $1.5\ 10^{10}$ |
| Ea UPN 527 | Ea PF 8 | $9.5\ 10^9$ | $8\ 10^{10}$ | $7.5\ 10^{10}$ |

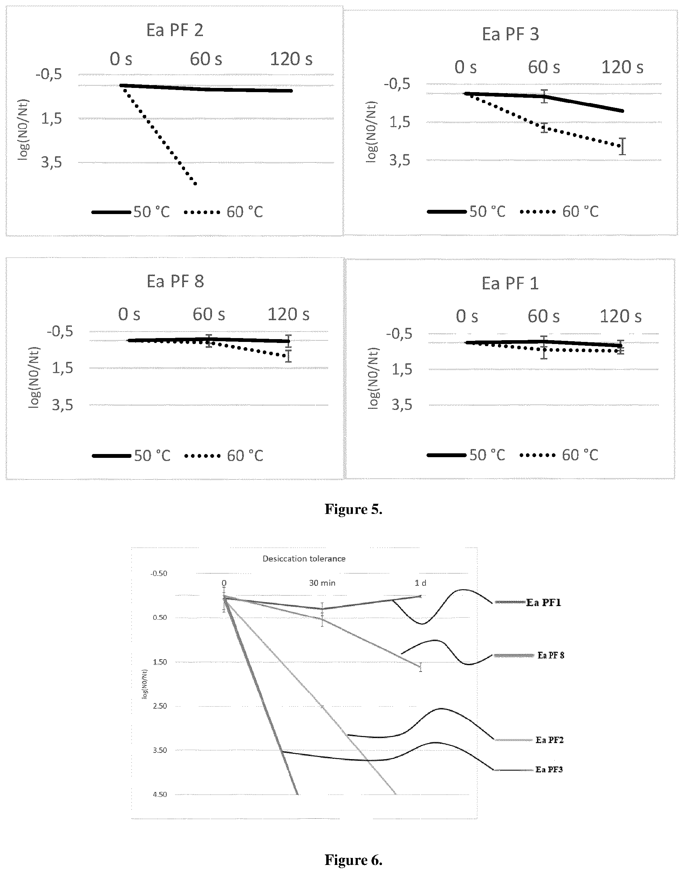

The phages contained in the cocktail are characterized by different tolerance toward heat and desiccation. At $50^0$C all phages showed good tolerance. It turned out to be the most resistant to $60^0$C Ea PF1 and Ea PF8, results for all phages are given on Figure 5. Phages Ea PF1 and Ea PF 8 seems are more stable to desiccation during 24 hours, see Figure 6.

Figure 5 shows the heat tolerance of phages at $50^0$C and $60^0$C

Figure 6 shows the desiccation tolerance of phages during 24 hours.

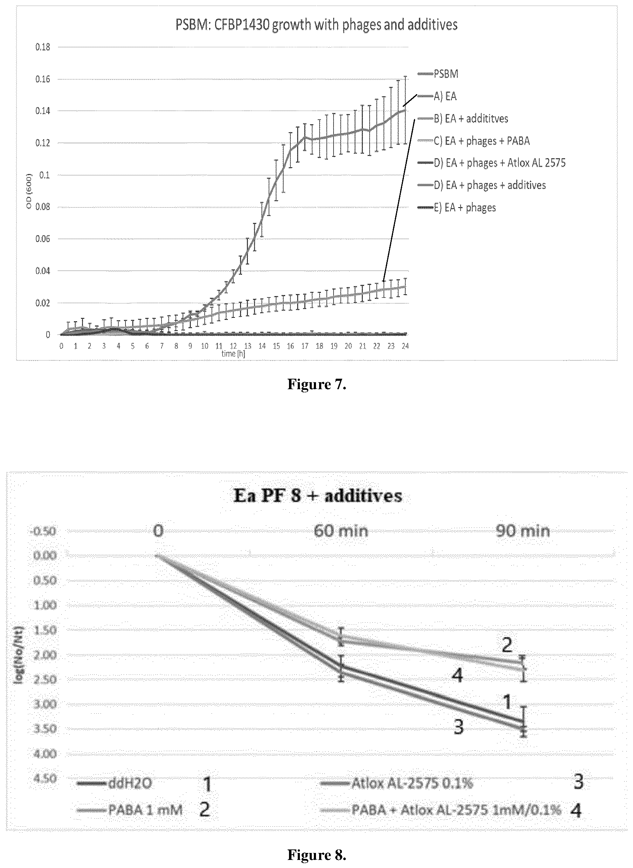

The phage cocktail was tested with additional product components UV protectant and surfactant: commercially available PABA and Atlox AL-2575 (alkyl polysaccharide). Growth curve assays were performed at 28°C and constant shaking in 96-well plates. PSBM (Pusey et al., 2011) served as the growth medium, as *E. amylovora* did not show any growth in the presence of PABA and Atlox AL-2575 in LB in previous experiments. *E. amylovora* CFBP1430 was present at $5\times10^5$ CFU/ml, and phages Ea PF 1, Ea PF 2, Ea PF 3 and Ea PF 8 in SM buffer were present at $10^6$ CFU/ml each. PABA was present at 1mM and Atlox AL-2575 at 0.1%. PABA was prepared in a stock of 8mM, and the pH was adjusted to 7 for solubility. Additives have some, but they do not have sufficient effect on bacterial growth and they do not affect the phage cocktail efficacy. Figure 7 shows the effect of the additives on the phage infectivity.

The product contains additives such as UV protectants and Surfactants to increase effectiveness and enhance the product against impact of the environmental factors. Based on the previous examinations, from phage cocktail was tested following combinations of the additives and phages (phage meaning the phage cocktail of PF-1, PF-2, PF-3 and PF-8 phages here): phage/distilled water, phage/PABA 1mM, phage/Atlox AL-2575 0.1% and phage/PABA/Atlox AL-2575 1mM/0.1%, i.e. component A, component B and component C). Surfactant has no effect against UV irradiation, but PABA was able to protect phages from UV irradiation as combined only with phage as well in combination with surfactant Atlox AL-2575.

Figure 8. shows the effect of the additives against UV irradiation.

**EXAMPLE 5- Green house experiment and field trials with the phage cocktail**

**Green house experiment**

A greenhouse experiment was performed for 20 days with 3 applications of the products.

Greenhouse experiment was carried out on apple trees, cultivar Topaz. Trees were divided into 5 groups, in each group was 14 plants: group 1 - negative control, group 2- positive control, group 3 - phage cocktail 1 (the cocktail of the invention), group 4 - phage cocktail 2, group 5 - Serenade. Based on host range results of the phages for test was chosen bacteria *Erwinia amylovora* CFBP 1430.

Before application on all trees was chosen 6 leaves to scratch it with special sterile paper, for each leaf was used new one, 84 leaves in each group. Both cocktails were diluted in following way: 978 ml of dis. water + 10 ml Component B + 10 ml Component C, was mixed well, then was added 2 ml of the component A and again mixed well. Then in application bottle was poured 90 ml of distilled (dis.) water + 10 ml of previously diluted new phage product and mixed well, total volume was 100 ml. 1 ml of Serenade was diluted in 99 ml of distilled water and mixed well, total volume was 100 ml.

On sufficient test groups was applied 100 ml of test products: phage cocktail 1(new), phage cocktail 2 (old) and Serenade. On positive and negative control groups was applied 100 ml of a distilled sterile water.

50 ml night culture of CFBP 1430 was centrifuged 3000 g for 30 minutes, supernatant was discarded, pellet was diluted with 40 ml PBS and measured OD= 1.5, was performed several dilutions to get OD=0.2, then was diluted $10\times$ times in 500 ml PBS. After 1 hour of the application of the test products, was applied 100 ml of bacterial suspension on al groups except negative control, on negative control was applied just sterile PBS 100 ml. From applied phage cocktails and bacteria was done sampling for quality control: 100 μl per samples. Phage cocktail samples were tested with double agar method, also was determined CFU for bacteria sample. Same way was done 2nd and 3rd application on after day 3 and 6 from first day of application. Plants was monitored after 1 week of application every 3 day, for evaluation of effect was determined following criteria: group 1 - was seen no infection on a leaf, group 2 - infected less than 50 % of a leaf, group 3 - infected 50-70 % of a leaf, group 4- infected whole area of a leaf and group 5 - when infection is turn into systematic character. Plants were monitored for 20 days every 3 days.

**Results with the greenhouse experiment**

The greenhouse experiment was performed for 20 days with 3 applications of the products. Monitoring started after 7 days of the first application of products, and after 10 days there was seen significant signs of the infection. Infection evaluation was divided into 5 categories: the first category was when there was seen as no infection, the second category was when less than 50% of the leaf was infected, the third category was when more than 50% of the leaf was infected, fourth category when more than 90% of the leaf was infected and fifth categories when there was seen systematic infection of the leaves. was counted a number of the leaves belonging to categories, results are described in Figure 9. As is can be seen from the results after 20 days phage cocktail 1, i.e the New cocktail (the cocktail of the invention) showed far the best results.

Figure 9 shows the comparative analysis of greenhouse experiment results for 10 and 20 days of monitoring. a. positive control of infection. b. Effect of the New cocktail on infection. c. Effect of the Old cocktail on infection. d.. Effect of the Serenade on infection.

**Field trials**

Was performed several Field trials to determine efficacy of the product for control Fire blight in pear and apples in Spain (4 locations), Portugal, Belgum, Greece and Romania. Climate Zone: EPOMED EPPO Mediterranean. GEP compliance was claimed in respect of this studies. EUROFINS AGROSCIENCE SERVICES is officially accredited to carry out efficacy testing in accordance with Commission Regulation (EU) N° 284/2013 by the relevant authorities in each country.

National regulatory guidelines were followed for the countries involved in the studies. EPPO guideline took precedence when required to ensure uniform program across Europe.

**Field Trial Results**

The Field Trial has been performed as described above with specific modifications in the various locations as mentioned below.

The Final reports with methodology, results and relevant certificates of entities are included as Appendixes.

**Field trial study in Portugal, Leiria, Vau.**

The following disease appeared at the trial site: Fireblight (Erwinia amylovora) - ERWIAM
The assessment was based on counting the number of affected flower clusters or shoot tips in three trees per plot. The affected shoots were removed after each assessment, so that the accumulated incidence was calculated for efficacies. On shoots, ERWIAM (Erwinia amylovora) was observed at 6 day after -A assessment timing, with a mean incidence of 6,8% in untreated plots. Until 12 day after -C, the disease levels increased up to 12.3% accumulated incidence in the untreated.

Under natural sourcing of the disease, moderate to good control was observed for both, medium dose of the test item applied at 0.1 L/ha and higher dose applied at 0.2 L/ha. Medium dose of the test item Phage Fire applied at 0.1 L/ha ranged from 56.5% at 6 day after -A up to 63.3% at 29 day after -C, performing competitive or even similarly to the standard Aliette Flash, applied at 3,75 kg/ha. No significant dose response relationship was notable and no statistically significant differences (p<0.5) were achieved.

**Field trial study in Spain, Zaragosa, Morata de Jiloca**

The following disease appeared at the trial site: Fireblight (Erwinia amylovora) - ERWIAM
The assessment was based on counting the number of affected flower clusters or shoot tips in three trees, per plot. The affected shoots were removed after each assessment, so that the accumulated incidence was calculated for efficacies. On shoots, ERWIAM (Erwinia amylovora) was observed at 6 days after -B assessment timing, with a mean incidence of 0,4% in untreated plots. Until 31 days after -C, the disease levels increased up to 1.4% accumulated incidence in the untreated.

Under natural sourcing of the disease, moderate to good control was observed for all doses of the test item applied. Highest dose of the test item Phage Fire applied at 0.2 L/ha reported 75,0% control during the whole trial conduct, performing better than the standard Aliette WG, applied at 0,3 kg/100 l. No significant dose response relationship was notable and no statistically significant differences (p<0.5) were achieved.

**Field trial study in Spain, Lleida, Soses**

The following disease appeared at the trial site: Fireblight (Erwinia amylovora) - ERWIAM
The assessment was based on counting the number of affected flower clusters or shoot tips in three trees, per plot. The affected shoots were removed after each assessment, so that the accumulated incidence was calculated for efficacies. On shoots, ERWIAM (Erwinia amylovora) was observed at 4 day after -A assessment timing, with a mean incidence of 3,0% in untreated plots. Until 32 day after -C, the disease levels increased up to 8,9% accumulated incidence in the untreated.

Under natural sourcing of the disease, good control was observed for both, medium dose of the test item applied at 0.1 L/ha and higher dose applied at 0.2 L/ha. Medium dose of the test item Phage Fire applied at 0.1 L/ha ranged from 70,4% at 32 day after -C up to 77,6% at 3 day after -B, and higest dose of the test item Phage Fire applied at 0.2 L/ha ranged from 70,9% at 12 day after -C up to 75,1% at 3 day after -B performing both, better than the standard Aliette WG, applied at 0,3 kg/100 l. Significant dose response relationship was notable with statically significant differences (p<0.5) at both 12 day after-C and 32 day after-C assessment timings.

**Field trial study in Spain, Navara, Sartaguda**

A bactericide trial was established in a representative apple grove in Sartaguda (Navarra) over one of the most sensitive cultivars and prior to Erwinia amylovora presence. Thus, the trial was commenced in a preventive way. Three foliar applications were performed during the flowering period and just one efficacy assessment timing carried out, 85 Day after C. Evaluations were done by counting the number of affected shoots and fruits per elementary plot, directly on the field. After this, the counted and affected shoots and fruits were removed to avoid a lack of control due to the deep capacity of the target disease propagation. So, final severity damage should be defined as the summation of affected shoots and fruits on the whole assessment program. However, according to the disease development, occurred during trial development, not a deep incidence of target disease was presence during trial development. Taking this into account, the current discussion will be based on the date recorded 85 day after C.

Efficacy results against *Erwinia amylovora* on shoots and fruits

Prior going into discussion, as we commented before, it should be necessary to clarify that finally, the presence of Erwinia amylovora during the trial course was found to be less representative than expected. Thus, data included on this report should be put in context, as the different products involved in the study were applied with a low disease level. Regarding severity, assessment was focused on the most representative part of the trees in which the different products should obtain the highest effects against target disease. Taking this into account, and refered to the summatory of final affected shoots, a clear trend seems to occur in the case of PHAGEFIRE, to the highest rate, controlling the target disease.

After this assessment, PHAGEFIRE, at highest rate and most preferred composition, displayed 50% of control, with just 0.3 affected shoots per plot and 100% of efficacy, in terms of fruits affected, with no damaged fruits. On the other hand, due to the low incidence found of target disease on Untreated, no efficacy may be attributed for lower rates of PHAGEFIRE or to the standard ALIETTE. (Figure 15)

Due to the final disease pressure, present across the trial, the different products performance should be understood more as a potential behaviour, rather than a conclusive one, since no significant threshold could be reached at the end. However, under the conditions of this trial, a reliable control against Erwinia amylovora could be attributed to PHAGEFIRE formulation, at higher rate, based on the data obtained on this study.

*Effects on non-target organisms*

No objective effects regarding non-target organisms, by visual assessment, could be recorded at any timing.

*Phytotoxicity*

No phytotoxicity symptoms have been noticed, at any timing, for none of the tested formulations

**Field trial study in Spain, Logrono, Rincon de soto**

A bactericide trial was established in a representative pear grove in Rincón de Soto (La Rioja) over one of the most sensitive cultivars and prior to Erwinia amylovora presence. Thus, the trial was commenced in a preventive way. Three foliar applications were performed during the first and principal flowering and three more during the second flowering period. In addition, just one assessment could be done, according to the disease development during trial execution. Evaluation was done by counting the number of affected shoots per elementary plot, directly on the field. After this, the counted and affected shoots were removed to avoid a lack of control due to the deep capacity of the target disease propagation. So, final severity damage should be defined as the summation of affected shoots on the whole assessment program. However, since just one assessment timing could be carried out, we will base this discussion on the results registered 33 DAF.

Efficacy results against Erwinia amylovora on shoots Prior going into discussion, it should be necessary to clarify that finally, the presence of Erwinia amylovora during the trial course was found to be less representative than expected. However, the obtained results, 33 DAF, allowed to define a clear control against target disease. Thus, data included on this report should be put in context, as the different products involved in the study were applied with not a high disease pressure.

Regarding severity, assessment was focused on the most representative part of the trees in which the different products should obtain the highest effects against target disease. Taking this into account, and refered to the summatory of final affected shoots, a clear effect seems to occur in the case of PHAGEFIRE, controlling the target disease. After this assessment, PHAGEFIRE, at medium and highest rates, displayed 83.9% and 77.4% of control, respectively, with just 1.25 and 1.75 affected shoots per plot. A clear rate response can be observed for this formulation, where the lower rate achieved a sensitively minor control than medium or higher. This lower rate displayed just 45.2% of control, and 4.25 affected shoots per plot, compared with Untreated, which showed 7.75 shoots per plot affected by target disease. The standard chosen (Fosetil-Al) managed to reduce significantly the disease pressure, compared with Untreated, displaying a efficacy of 71% and 2.25 shoots affected per plot.

Under the conditions of this trial, PHAGEFIRE at 0.1 L/ha, showed to be the minimum effective dose controlling the target disease. Due to the final disease pressure, present across the trial, the different products performance should be understood reliable for the study purpose, since a significant disease presence occurred 33 Day after F.

*Effects on non-target organisms*

No objective effects regarding non-target organisms, by visual assessment, could be recorded at any timing.

*Phytotoxicity*

No phytotoxicity symptoms have been noticed, at any timing, for none of the tested formulations

### Field trial study in Greece, Pells 59, Giannitsa

The following disease appeared at the trial site: Fireblight (Erwinia amylovora) - ERWIAM
The assessment was based on counting the number of affected flower clusters or shoot tips in three trees, per plot. The affected shoots were removed after each assessment, so that the accumulated incidence was calculated for efficacies. On shoots, ERWIAM (Erwinia amylovora) was observed at 10 day after-F assessment timing, with a mean incidence of 8.0% in untreated plots. Until 20 day after-F, the disease levels increased up to 21.3% accumulated incidence in the untreated.

Under natural sourcing of the disease, good control was observed for both, medium dose of the test item applied at 0.1 L/ha and higher dose applied at 0.2 L/ha. Higher dose of the test item Phage Fire applied at 0.2 L/ha ranged from 83.0% at 10 day after-F up to 84.9% at 20 day after-F, performing competitive or even similarly to the standard Aliette 80 WG, applied at 3,75 kg/ha. Significant dose response relationship was notable with statically significant differences (p<0.5) were achieved.

### Field trial study in Belgum, Limburg, Sint-Truiden

The following disease was artificially inoculated at the trial site: Fireblight (Erwinia amylovora) - ERWIAM The inoculation was made at full flowering, 2 DA- B and 3 DB-C, by spraying a liquid bacterial solution on 30 flower cluster per tree.

The assessment was based on scoring, according to a 0-4 scale for necrosis and to a 0-3 scale for ooze droplet formation at flower level, each individual flower per cluster.

The target disease, ERWIAM (Erwinia amylovora) was observed at 4 DA-C assessment timing, with a mean necrosis incidence on flowers of 39,9% and 20,6% infection degree (Severity) in untreated plots. Until 10 DAC, the disease levels increased up to a mean necrosis incidence on flowers of 96,1% and 90,4% infection degree (Severity) in untreated plots.

Regarding to the ooze droplet formation at flower level, it was observed 8,5% incidence and 3,3% infection degree (Severity) at 4 DA-C increasing up to 13,5% incidence and 6,6% infection degree (severity) at 7 DA-C, in the untreated plots.

Under artificial sourcing of the disease, causing a high infection, no significant control was observed in necrosis in flowers. By contrast, moderate to good control was observed all dose of the test item related to both, ooze droplet formation incidence and ooze infection degree, ranging values from 47,5% to 68,8% incidence control and from 50,8% to 70,9% ooze infection degree control at 7 DA-C assessment timing, performing most of the cases better than the standard Aliette WG, applied at 2,5 kg/ha.

No significant dose response relationship was notable and no statically significant differences (p<0.5) were achieved.

**Field trial study in Romania, RIFG Pitesti-Maracineni**

In 2023, the local conditions seem to be good for fire blight infections and disease development.

The studied variety "Topaz" reveal that it behaves like sensitive according its genetic background, treatments applied during the vegetation season and the evaluations moment well.

PHAGEFIRE applied at a rate of 0.1 L/ha can offer a fair protection against fire blight infections for sensitive apple varieties if infection pressure is low.

A deep look on the Figure 10 reveals that the product PHAGEFIRE protected very well the "Topaz" apple fruits against infections with *Erwinia amylovora* bacteria. In the variant with Product PHAGEFIRE applied at a rate of 0.1L/ha, the efficacy [E abbot %] was 95.00. In the variant with Product PHAGEFIRE applied at a rate of 0.2L/ha, the efficacy [E abbot %] was 100.00. By comparation, in the variant treated with Serenade ASO 6.00 L/ha the efficacy [E abbot %] was 95.00-96.88 and, in the variant treated with Aliette 80 WG 3.75 kg/ha the efficacy [E abbot %] was 93.75-100.

In more somewhat more detail, the assessment of the Figure 10.a reveals that the product protected very well the 'Topaz' apple blossoms against the early infections with Erwinia amylovora bacteria. In particular the variant applied at a rate of 0.2 L/ha, the efficacy [E Abbott %] was 100. By comparation, in the variant treated with Serenade ASO 6.00 l/ha the efficacy [E Abbott %] was 94.52. Similarly, Figures 10.b and 10.c show that the product protected very well the 'Topaz' apple shoots. In particular, the product applied at a rate of 0.2 L/ha, the efficacy [E Abbott %] was 100. By comparation, in the variant treated with Serenade ASO 6.00 l/ha the efficacy [E Abbott %] was 83.33. Furthermore, the cocktail product of the invention protected very well the 'Topaz' apple fruits, in particular, if applied at a rate of 0.2 L/ha, the efficacy [E Abbott %] was 100. By comparation, in the variant treated with Serenade ASO 6.00 l/ha the efficacy [E Abbott %] was 95.00-96.88.

Figure 10.a shows the overall efficacy of the product in protecting "Topaz" apple trees on spurs, shoots and fruits against fire blight.

In conclusion, while in 2023 the local conditions seems to be good for fire blight infections and disease development, and the studied variety 'Topaz' was sensitive according its genetic background, treatments applied during the vegetation seasons provided protection; in particular when it was applied at a rate of 0.1 L/ha it can offer a fair protection against fireblight infections, similar to prior art methods, whereas at a rate of 0.2 L/ha it proved to be significantly better than the art treatments.

INDUSTRIAL APPLICABILITY

The phages for the cocktail were chosen based on the host range results, but because of their diverse spectrum of activity, when combined in the cocktail they showed a synergistic effect against a world wild spectrum of bacteria, even the least susceptible ones, which is a great advantage of cocktail use.

Excipients such as commercially available PABA and Atlox AL-2575 (alkyl polysaccharide) made the product more resistant to UV and desiccation, but did not affect its effectiveness toward bacteria.

Greenhouse experiment and several field trials were to determine efficacy of the product for control Fire blight in pear and apples in Spain (4 locations), Portugal, Belgum, Greece and Romania. Climate Zone: EPOMED EPPO Mediterranean.

Based on results of the field trials we can conclude as follows:

Excellent results of product formulation at medium and higher rates compared to untreted plots.

No phytotoxicity found for ant of the tested products.

**Claims**

1. A biopesticide composition comprising
a) at least one bacteriophage capable of infecting or lysing Erwinia sp bacteria, b) an UV protectant compound, and c) a surfactant compound and optionally d) further excipient compounds.

2. The biopesticide composition according to claim 1, wherein

a) the at least one bacteriophage capable of infecting or lysing Erwinia sp bacteria is effective against *Erwinia amylovora,* preferably effectively lyses more than one *Erwinia amylovora* strains,
b) the UV protectant compound is an aminobenzoate UV-protectant, and
c) the surfactant compound is a polysaccharide compound, preferably an alkyl polysaccharide.

3. The biopesticide composition (preferably a biopesticide composition according to any of the previous claims), wherein the one or more bacteriophage is/are capable of infecting or lysing *Erwinia amylovora,*
said bacteriophage selected from the group consisting of

bacteriophage (Ea PF1) deposited accession number DSM 34958, or a derivative, variant or mutant thereof with a genomic sequence at least 90% identical to the sequence of SEQ ID NO: 1;
bacteriophage (Ea PF2) deposited on under accession number DSM 34959, or a derivative, variant or mutant thereof with a genomic sequence at least 90% identical to the sequence of SEQ ID NO: 2;
bacteriophage (Ea PF3) deposited on under accession number DSM 34960, or a derivative, variant or mutant thereof with a genomic sequence at least 90% identical to the sequence of SEQ ID NO: 3;
bacteriophage (Ea PF8) deposited on under accession number DSM 34961, or a derivative, variant or mutant thereof with a genomic sequence at least 80% identical to the sequence of SEQ ID NO: 4;
each derivative, variant or mutant being capable of infecting/lysing *Erwinia amylovora,*
and an auxiliary material, preferably an agricultural excipient, wherein preferably the composition comprises more than one isolated bacteriophage capable of infecting or lysing *Erwinia amylovora.*

4. The biopesticide composition according to claim 3, comprising

bacteriophage (Ea PF1) deposited under accession number DSM 34958, or a derivative, variant or mutant thereof with a genomic sequence at least 90% identical to the sequence of SEQ ID NO: 1;
bacteriophage (Ea PF2) deposited under accession number DSM 34959, or a derivative, variant or mutant thereof with a genomic sequence at least 90% identical to the sequence of SEQ ID NO: 2;
bacteriophage (Ea PF3) deposited under accession number DSM 34960, or a derivative, variant or mutant thereof with a genomic sequence at least 90% identical to the sequence of SEQ ID NO: 3;
bacteriophage (Ea PF8) deposited under accession number DSM 34961, or a derivative, variant or mutant thereof with a genomic sequence at least 80% identical to the sequence of SEQ ID NO: 4;
each derivative, variant or mutant being capable of infecting/lysing *Erwinia amylovora.*

5. The biopesticide composition according to any of claims 1 to 4, preferably 3 to 4 or 4, capable of lysing at least one, preferably at least 2, more preferably at least 10 of the following *Erwinia amylovora* strains listed in table 1 or table 4, and/or, effective against an *Erwinia amylovora* strain selected from *Erwinia amylovora* CFBP 1430 = DSM 34956

*Erwinia amylovora* OR-25 = DSM 34957, and/or
capable of lysing at least one, preferably at least 2, more preferably at least 10 of the following *Erwinia amylovora* strains listed in table 2.

6. The biopesticide composition according to any of claims 3 to 5, wherein, preferably measured with spot test,

- phage Ea PF1 or its derivative, variant or mutant is capable of lysis of at least 90% of the Ea. strains of table 4, preferably confluent lysis of at least 50%, preferably at least 70 % of the Ea. strains of table 4,
- phage Ea PF 2 or its derivative, variant or mutant is capable of lysis of at least 50%, preferably at least 60 % of the Ea. strains of table 4, preferably confluent lysis of at least 25% of the Ea. strains of table 4,
- phage Ea PF 3 or its derivative, variant or mutant is capable of lysis of at least 60%, preferably at least 70 % of the Ea. strains of table 4, preferably confluent lysis of at least 25% of the Ea. strains of table 4,
- phage Ea PF 8 or its derivative, variant or mutant is capable of lysis of at least 90%, preferably 95%, highly preferably 100% of bacteria, preferably confluent lysis of at least 30%, preferably at least 35 % of the Ea. strains of table 4,

7. The biopesticide composition according to any of claims 3 to 6, wherein

- phage Ea PF 1 or its derivative, variant or mutant is capable to produce $10^{11}$ genome copies/ml in the case of at least 60%, preferably in 73% of the tested bacteria,
- phage Ea PF 2 or its derivative, variant or mutant is capable to produce $10^{11}$ genome copies/ml in the case of at least 12%, preferably in 16% of the tested bacteria,
- phage Ea PF 3 or its derivative, variant or mutant is capable to produce $10^{11}$ genome copies/ml in the case of at least 12%, preferably in 16% of the tested bacteria,
- phage Ea PF 8 or its derivative, variant or mutant is capable to produce $10^{11}$ genome copies/ml in the case of at least 60%, preferably in 70% of the tested bacteria,

if measured by QPCR.

8. The biopesticide composition according to any of claims 3 to 7, wherein

   each of the phages are heat tolerant up to 50°C,
   wherein preferably Ea PF1 and Ea PF8 are heat tolerant up to 60°C, and/or
   wherein preferably Ea PF1 and Ea PF8 are tolerant to desiccation during 24 hours.

9. A cocktail of bacteriophages comprising two or more bacteriophages selected from the bacteriophages as defined in any of claims 3 to 8, and a biologically, preferably agronomically tolerable excipient.

10. The cocktail of bacteriophages according to claim 9, said cocktail comprising

    bacteriophage (Ea PF1) deposited under accession number DSM 34958, or a derivative, variant or mutant thereof with a genomic sequence at least 90% identical to the sequence of SEQ ID NO: 1;
    bacteriophage (Ea PF2) deposited under accession number DSM 34959, or a derivative, variant or mutant thereof with a genomic sequence at least 90% identical to the sequence of SEQ ID NO: 2;
    bacteriophage (Ea PF3) deposited under accession number DSM 34960, or a derivative, variant or mutant thereof with a genomic sequence at least 90% identical to the sequence of SEQ ID NO: 3; and
    bacteriophage (Ea PF8) deposited under accession number DSM 34961, or a derivative, variant or mutant thereof with a genomic sequence at least 80% identical to the sequence of SEQ ID NO: 4;
    each derivative, variant or mutant being capable of infecting *Erwinia amylovora.*

11. A bacteriophage capable of infecting/lysing *Erwinia amylovora* said bacteriophage selected from the group consisting of

    bacteriophage (Ea PF1) deposited under accession number DSM 34958, or a derivative, variant or mutant thereof with a genomic sequence at least 90% identical to the sequence of SEQ ID NO: 1;
    bacteriophage (Ea PF2) deposited under accession number DSM 34959, or a derivative, variant or mutant thereof with a genomic sequence at least 90% identical to the sequence of SEQ ID NO: 2;
    bacteriophage (Ea PF3) deposited under accession number DSM 34960, or a derivative, variant or mutant thereof with a genomic sequence at least 90% identical to the sequence of SEQ ID NO: 3;
    bacteriophage (Ea PF8) deposited under accession number DSM 34961, or a derivative, variant or mutant thereof with a genomic sequence at least 80% identical to the sequence of SEQ ID NO: 4;
    each derivative, variant or mutant being capable of infecting *Erwinia amylovora.*

12. An isolated bacteriophage or the cocktail of isolated bacteriophages capable of infecting/lysing *Erwinia amylovora* said bacteriophage selected from the group consisting of bacteriophage (Ea PF8) deposited under accession number DSM 34961, or a derivative, variant or mutant thereof with a genomic sequence of at least 80%, preferably of at least 90%, more preferably 95% identical to the sequence of SEQ ID NO: 1.

13. The isolated bacteriophage or the cocktail according to claim 12 capable of lysing activity on at least 90% of the *Erwinia amylovora* strains listed in Table 4.

14. The isolated bacteriophage or the cocktail according to claim 13, wherein confluent lysis (4+) is present in at least 30, preferably at least 40% of the bacteria.

15. The isolated bacteriophage or the cocktail according to any of claims 13 to 14, wherein the phage is able to produce $10^{11}$ genome copies/ml in the case of 70% of the tested bacteria as shown by QPCR.

16. The isolated bacteriophage or the cocktail according to any of claims 13 to 15 wherein the Ea PF 8 phage is effective and can produce more than $10^9$ genome copies/ml in 67% of tested bacteria strains in Table 2.

17. The isolated bacteriophage or the cocktail according to any of claims 12 to 16, wherein said bacteriophage is heat tolerant, preferably tolerant at 50°C for at least 2 minutes, preferably according to the measurement described in the materials and methods.

18. The isolated bacteriophage or the cocktail according to any of claims 12 to 17, said phage being desiccation tolerant for at least 24 hours.

19. A use of the isolated bacteriophage or the cocktail according to any of claims 12 to 18 for the control of *Erwinia amylovora.*

20. The use of the isolated bacteriophage or the cocktail according to claim 19, to a plant, preferably a Rosaceae plant, to prevent or treat bacterial infection caused by *Erwinia amylovora.*

21. A use of the biopesticide composition according to claim any of claims 1 to 8 or a cocktail of bacteriophages according to any of claims 10 to 11, for the control of *Erwinia amylovora.*

22. The use of the isolated bacteriophage or the cocktail according to claim 21, to a plant, preferably a Rosaceae plant, to prevent or treat bacterial infection caused by *Erwinia amylovora.*

23. A method for the control of *Erwinia amylovora,* comprising the steps of application of a bacteriophage according to any of claims 11 to 20 or of a biopesticide preparation according to any of claims 1 to 8 or a cocktail of bacteriophages according to any of claims 9 to 10.

24. The method according to claim 23, to prevent or treat bacterial infection caused by *Erwinia amylovora* in a plant, preferably in a Rosaceae plant, said method comprising the step of contacting the plant with the biopesticide composition according to any of claims 1 to 8 or a cocktail according to any of claims 9 to 10 or the bacteriophage according to any of claims 11 to 20.

25. The method according to claim 23 or 24, wherein

the leaves or the shoots of the plant are contacted at least one time, or preferably
at least two times within 30 days, or preferably
at least three times within 20 days.

26. The method according to claim 23 to 25 wherein said plant is a fruit tree plant of the Rosaceae family, preferably said plant is selected from apples, pears and quince.

**FIGURE 1**

Phage protein
Phage protein
Phage DNA helicase

DNA polymerase I (EC 2.7.7.7), p...

Phage protein

Phage-associated DNA primase

Phage protein
tRNA-Arg-GTT
tRNA-Asp-GTC
tRNA-Gln-TTG
tRNA-Ile-CAT
tRNA-Tyr-GTA
tRNA-Lys-TTT

Phage protein

Phage riIA lysis inhibitor
Methyl-directed repair DNA adeni...
Phage protein
Phage protein

Phage protein

RNA polymerase (EC 2.7.7.6), pha...
Phage protein

Phage integrase

Phage protein (ACLAME 769)

Phage terminase, large subunit

Cellulose 1,4-beta-cellobiosidas...
Phage tail length tape-measure p...
Phage secretion activator protei...
Phage protein
Phage portal (connector) protein
Phage tape measure protein

Seq59_P8_NODE_1_
75,270bp

70000
5000
65000
10000
60000
15000
55000
20000
50000
25000
45000
30000
40000
35000

**FIGURE 2.a**

39

**Figure.2b**

**Figure 3.a.**                                          **Figure 3b.**

Figure. 4

**Figure 5.**

**Figure 6.**

**Figure 7.**

**Figure 8.**

**Figure 9.**

**Figure 10.a**

**Figure 10.b**

**Figure 10.c**

**Figure 10.d**

**Figure 10.e**

**Figure 10.f**

PF-1

**Figure 11.**

PF-2

**Figure 12**

PF-3

**Figure 13**

PF-8

**Figure 14**

| | Europäisches Patentamt European Patent Office Office européen des brevets | **EUROPEAN SEARCH REPORT** | Application Number EP 24 16 7956 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | GAYDER STEVEN ET AL: "Biological control of the fire blight pathogen Erwinia amylovora using bacteriophages", JOURNAL OF PLANT PATHOLOGY, vol. 106, no. 3, 17 August 2023 (2023-08-17), pages 853-869, XP093215761, ISSN: 2239-7264, DOI: 10.1007/s42161-023-01478-y Retrieved from the Internet: URL:https://link.springer.com/content/pdf/10.1007/s42161-023-01478-y.pdf> * abstract * * page 857, left-hand column * * page 861, left-hand column, paragraph middle * * table 1 * ----- -/-- | 1,2,23 | INV. A01N63/40 A01P1/00 C12N7/00 |

TECHNICAL FIELDS SEARCHED (IPC)

C07K
A01N
A01P
C12N

The present search report has been drawn up for all claims

| Place of search | Date of completion of the search | Examiner |
|---|---|---|
| Munich | 18 October 2024 | Galley, Carl |

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JO SU JIN ET AL: "Optimizing the formulation of Erwinia bacteriophages for improved UV stability and adsorption on apple leaves", HELIYON, vol. 9, no. 11, 1 November 2023 (2023-11-01), page e22034, XP093215768, GB ISSN: 2405-8440, DOI: 10.1016/j.heliyon.2023.e22034 Retrieved from the Internet: URL:https://pdf.sciencedirectassets.com/31 3379/1-s2.0-S2405844023X00127/1-s2.0-S2405 844023092423/main.pdf?X-Amz-Security-Token =IQoJb3JpZ2luX2VjEMD//////////wEaCXVzLWVhc 3QtMSJGMEQCIEYAb1BJ9XouLk6UZxWwHLQMWUG1LJJ o7WufKLdM/nmSAiB8khto7oUePzfWOg/zAQFpMZu1Y cIojyXf2dN054++LiqzBQgpEAUaDDA1OTAwMzU0Njg 2NSIMVEkpB> * abstract * * Section 2.2; page 2 * * Section 2.3; page 3 * ----- -/-- | 1,2,23 | |
|  |  |  | TECHNICAL FIELDS SEARCHED (IPC) |

The present search report has been drawn up for all claims

| Place of search | Date of completion of the search | Examiner |
|---|---|---|
| Munich | 18 October 2024 | Galley, Carl |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

EP 24 16 7956

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | BORN YANNICK ET AL: "Novel Virulent and Broad-Host-Range Erwinia amylovora Bacteriophages Reveal a High Degree of Mosaicism and a Relationship to Enterobacteriaceae Phages", APPLIED AND ENVIRONMENTAL MICROBIOLOGY, vol. 77, no. 17, 1 September 2011 (2011-09-01), pages 5945-5954, XP093215577, US ISSN: 0099-2240, DOI: 10.1128/AEM.03022-10 Retrieved from the Internet: URL:https://journals.asm.org/doi/pdf/10.1128/AEM.03022-10> | 3-15,19, 23 | |
| Y | * abstract * * table 1 * * vB_EamM-Y2 and vB_EamP-L1 * * figure 7 * | 9,10 | |
| X | ESPLIN IAN N.D. ET AL: "Genome sequences of 19 novel Erwinia amylovora bacteriophages", GENOME ANNOUNCEMENTS, vol. 5, no. 46, 1 January 2017 (2017-01-01), XP093215825, US ISSN: 2169-8287, DOI: 10.1128/genomeA.00931-17 Retrieved from the Internet: URL:https://pmc.ncbi.nlm.nih.gov/articles/PMC5690319/pdf/e00931-17.pdf> | 3-8, 11-15, 19,23 | TECHNICAL FIELDS SEARCHED (IPC) |
| Y | * abstract * * vB_EamM_Parshik, vB_EamM_Huxley, vB_EamM_Machina; table 1 * | 9,10 | |

-----

-/--

The present search report has been drawn up for all claims

| Place of search | Date of completion of the search | Examiner |
|---|---|---|
| Munich | 18 October 2024 | Galley, Carl |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PARK JUNGKUM ET AL: "Characterization of the Lytic Bacteriophage phiEaP-8 Effective against Both Erwinia amylovora and Erwinia pyrifoliae Causing Severe Diseases in Apple and Pear", PLANT PATHOLOGY JOURNAL, vol. 34, no. 5, 1 October 2018 (2018-10-01), pages 445-450, XP093215830, KR ISSN: 1598-2254, DOI: 10.5423/PPJ.NT.06.2018.0100 Retrieved from the Internet: URL:https://ppjonline.org/upload/pdf/ppj-34-445.pdf> | 3-8, 11-15, 19,23 | |
| Y | * abstract * * phiEaP-8 * * table 1 * | 9,10 | |
| Y,D | BIOSCA ELENA G. ET AL: "First European Erwinia amylovora Lytic Bacteriophage Cocktails Effective in the Host: Characterization and Prospects for Fire Blight Biocontrol", BIOLOGY, vol. 13, no. 3, 8 March 2024 (2024-03-08), page 176, XP093215775, CH ISSN: 2079-7737, DOI: 10.3390/biology13030176 Retrieved from the Internet: URL:https://www.mdpi.com/2079-7737/13/3/176/pdf> * abstract * * page 2, paragraph final * | 9,10 | TECHNICAL FIELDS SEARCHED (IPC) |

The present search report has been drawn up for all claims

| Place of search | Date of completion of the search | Examiner |
|---|---|---|
| Munich | 18 October 2024 | Galley, Carl |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...........................................................................................
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020230087771
- KR 1020230087770 A
- CN 117363583 A
- KR 102025403 B1
- KR 1020230172817 A
- KR 1020230155980 A

- KR 1020220090446 A
- KR 1020230171294 A
- KR 1020230171303 A
- KR 1020230171302 A
- KR 101601912 B1

### Non-patent literature cited in the description

- Genetic diversity and host range of Erwinia amylovora. **MOMOL, M. T.** ; **ALDWINCKLE, H. S.** CABI Books.. CABI, 2000
- **HEVESI, M.** *Az Erwinia amylovora (Burill)*, 1996
- **WINSLOW et al.** hazai megjelenése almán. *Növényvédelem*, vol. 32 (5), 225-228
- **VÉGH, A.** ; **HEVESI, M.** ; **PÁJTLI, E. et al.** Characterization of Erwinia amylovora strains from Hungary.. *Eur J Plant Pathol*, 2017, vol. 147, 455-461
- Migration of Erwinia amylovora in host plant tissues. **VANNESTE, J.L.** ; **EDEN-GREEN, S.** Fire Blight: The Disease and its Causative Agent. CAB International, 2000, 73-83
- **SKONECZNY, H.** ; **KUBIAK, K.** ; **SPIRALSKI, M.** ; **KOTLARZ, J.** ; **MIKICIŃSKI, A.** ; **PULAWSKA, J.** Fire Blight Disease Detection for Apple Trees: Hyperspectral Analysis of Healthy. *Infected and Dry Leaves. Remote Sens.*, 2020, vol. 12, 2101
- **ZUM, J.D.** ; **NORELLI, J.L.** ; **MONTANARI, S.** ; **BELL, R.** ; **BASSIL, N.V.** Dissecting Genetic Resistance to Fire Blight in Three Pear Populations.. *Phytopathology*, 2020, vol. 110, 1305-1311
- **ESCURSELL, M.M.** ; **ROSCHI, A.** ; **SMITS, T.H.M.** ; **REZZONICO, F.** Characterization and Direct Molecular Discrimination of RpsL Mutations Leading to High Streptomycin Resistance in Erwinia Amylovora.. *J Plant Pathol*, 2021, vol. 103, 99-108
- **SEED, K. D.** Battling phages: how bacteria defend against viral attack.. *PLoS Pathog.*, 2015, vol. 11, e1004847
- **GOODRIDGE, L.D.** ; **ABEDON, S.T.** Bacteriophage Biocontrol: The Technology Matures.. *Microbiol. Aust.*, 2008, vol. 29, 48-49
- **POLASKA, M** ; **SOKOLOWSKA, B.** Bacteriophages-A New Hope or a Huge Problem in the Food Industry.. *AIMS Microbiol.*, 2019, vol. 5, 324

- **SIEIRO, C.** ; **AREAL-HERMIDA, L. et al.** A hundred years of bacteriophages: Can phages replace antibiotics in agriculture and aquaculture?. *Antibiotics*, 2020, vol. 9, 493
- **SVIRCEV, A.** ; **ROACH, D.** ; **CASTLE, A.** Framing the future with bacteriophages in agriculture.. *Viruses*, 2018, vol. 10, 218
- **JAMAL, M.** ; **BUKHARI, S.M.A.U.S. et al.** Bacteriophages: An overview of the control strategies against multiple bacterial infections in different fields.. *J. Basic Microbiol.*, 2019, vol. 59, 123-133
- **DÖMÖTÖR, D.** ; **BECSÁGH, P.** ; **RÁKHELY, G.** ; **SCHNEIDER, G.** ; **KOVÁCS,T.** Complete genomic sequence of Erwinia amylovora phage PhiEaH2. *Journal of Virology*, 2012, vol. 86 (19), 10899-10899
- **MECZKER, K.** ; **DÖMÖTÖR, D.** ; **VASS, J.** ; **RÁKHELY, G.** ; **SCHNEIDER, G.** ; **KOVÁCS, T.** The genome of the Erwinia amylovora phage PhiEaH1 reveals greater diversity and broadens the applicability of phages for the treatment of fire blight.. *FEMS Microbiology Letters*, 2014, vol. 350 (1), 25-27
- **SCHWARCZINGER, I;** ; **KOLOZSVÁRINÉ NAGY, J. et al.** Characterization of Myoviridae and Podoviridae family bacteriophages of Erwinia amylovora from Hungary-potential of application in biological control of fire blight.. *European Journal of Plant Pathology*, 2017, vol. 149 (3), 639-652
- **KIM, H.J** ; **JUN, J.W. et al.** Bacteriophage cocktail for the prevention of multiple-antibiotic-resistant and mono-phage-resistant Vibrio coralliilyticus infection in pacific oyster (Crassostrea gigas)larvae.. *Pathogens*, 2020, vol. 9, 831
- **KIM, S.G.** ; **LEE, S.B. et al.** Phage cocktail in combination with kasugamycin as a potential treatment for fire blight caused by Erwinia amylovora.. *Antibiotics*, 2022, vol. 11, 1566

- **GILL, J. J. et al.** Bacteriophages of Erwinia amylovora. *Applied and Environmental Microbiology*, April 2003, 2133-2138
- **BOULÉ et al.** Isolation and characterization of eight bacteriophages infecting Erwinia amylovora and their potential as biological control agents in British Columbia, Canada. *Can. J. Plant Pathol.*, 2011, vol. 33 (3), 308-317
- **KNECHT LE** ; **BORN Y** ; **PELLUDAT C** ; **POTHIER JF** ; **SMITS THM** ; **LOESSNER MJ** ; **FIESELER L.** Spontaneous Resistance of Erwinia amylovora Against Bacteriophage Y2 Affects Infectivity of Multiple Phages.. *Front. Microbiol.*, 2022, vol. 13, 908346
- **THOMPSON, DANIEL W. et al.** Genomic comparison of 60 completely sequenced bacteriophages that infect Erwinia and/or Pantoea bacteria. *Virology*, 2019, vol. 535, 59-73
- **PARK J** ; **LEE GM** ; **KIM D** ; **PARK DH** ; **OH CS.** Characterization of the Lytic Bacteriophage phiEaP-8 Effective against Both Erwinia amylovora and Erwinia pyrifoliae Causing Severe Diseases in Apple and Pear.. *Plant Pathol J.*, 01 October 2018, vol. 34 (5), 445-450
- **GAYDER, S.** ; **KAMMERECKER, S.** ; **FIESELER, L.** Biological control of the fire blight pathogen Erwinia amylovora using bacteriophages.. *J Plant Pathol*, 2023
- **BIOSCA EG** ; **DELGADO SANTANDER R** ; **MORÁN F** ; **FIGÀS-SEGURA À** ; **VÁZQUEZ R** ; **CATALÀ-SENENT JF** ; **ALVAREZ B.** First European Erwinia amylovora Lytic Bacteriophage Cocktails Effective in the Host: Characterization and Prospects for Fire Blight Biocontrol.. *Biology.*, 2024, vol. 13 (3), 176
- **BORN Y** ; **FIESELER L** ; **MARAZZI J** ; **LURZ R** ; **DUFFY B** ; **LOESSNER MJ.** Novel virulent and broad-host-range Erwinia amylovora bacteriophages reveal a high degree of mosaicism and a relationship to Enterobacteriaceae phages.. *Appl Environ Microbiol.*, September 2011, vol. 77 (17), 5945-54
- **KIM, H.J.** ; **JUN, J.W.** ; **GIRI, S.S.** ; **KIM, S.G.** ; **KIM, S.W.** ; **KWON, J.** ; **LEE, S.B.** ; **CHI, C.** ; **PARK, S.C.** Bacteriophage cocktail for the prevention of multiple-antibiotic-resistant and mono-phage-resistant Vibrio coralliilyticus infection in pacific oyster (Crassostrea gigas) larvae.. *Pathogens*, 2020, vol. 9, 831. 24
- **KIM, S.G** ; **LEE, S.B.** ; **JO, S.J.** ; **CHO, K.** ; **PARK, J.K.;** ; **KWON, J.** ; **GIRI, S.S.** ; **KIM, S.W** ; **KANG, J.W.** ; **JUNG, W.J.; et al.** Phage cocktail in combination with kasugamycin as a potential treatment for fire blight caused by Erwinia amylovora.. *Antibiotics*, 2022, vol. 11, 1566
- **BIOSCA EG** ; **DELGADO SANTANDER R et al.** First European Erwinia amylovora Lytic Bacteriophage Cocktails Effective in the Host: Characterization and Prospects for Fire Blight Biocontrol.. *Biology.*, 2024, vol. 13 (3), 176
- **SMITH, HAMILTON O.** Clyde A.. *Hutchison*, vol. III
- **CYNTHIA PFANNKOCH** ; **J. CRAIG.** *Venter PNAS*, 23 December 2003, vol. 100 (26), 15440-15445
- **HUANG-JIE.** Front. Microbiol.. *Sec. Phage Biology*, 31 May 2023, vol. 14, 2023
- **SUN Q et al.** Advance on Engineering of Bacteriophages by Synthetic Biology.. *Infect Drug Resist.*, 2023, vol. 16, 1941-1953
- **GARENNE, DAVID et al.** *Current Opinion in Systems Biology*, 2021, vol. 28, 100373
- **GILL, J. J.** ; **SVIRCEV, A. M** ; **SMITH, R.** ; **CASTLE, A. J.** Bacteriophages of Erwinia amylovora.. *Applied and Environmental Microbiology*, 2003, vol. 69 (4), 2133-2138
- **ADAMS, M.H.** Bacteriophages.. Inter Science Publishers, 1959, vol. 27
- **KUTTER E.** Phage host range and efficiency of plating.. *Methods Mol Biol.*, 2009, vol. 501, 141-9
- **KROPINSKI AM** ; **MAZZOCCO A** ; **WADDELL TE** ; **LINGOHR E** ; **JOHNSON RP.** Enumeration of bacteriophages by double agar overlay plaque assay.. *Methods Mol Biol.*, 2009, vol. 501, 69-76
- **GAYDER S** ; **PARCEY M** ; **CASTLE AJ** ; **SVIRCEV AM.** Host Range of Bacteriophages Against a World-Wide Collection of Erwinia amylovora Determined Using a Quantitative PCR Assay.. *Viruses*, 01 October 2019, vol. 11 (10), 910
- **KROPINSKI AM** ; **MAZZOCCO A** ; **WADDELL TE** ; **LINGOHR E** ; **JOHNSON RP.** Enumeration of bacteriophages by double agar overlay plaque assay. *Methods Mol Biol.*, 2009, vol. 501, 69-76
- **DUARTE J** ; **PEREIRA C.** Bacteriophages with Potential to Inactivate Aeromonas hydrophila in Cockles: In Vitro and In Vivo Preliminary Studies.. *Antibiotics (Basel)*, 12 June 2021, vol. 10 (6), 710
- **KIM, S.G.** ; **JUN, J.W.** ; **GIRI, S.S.** ; **YUN, S** ; **KIM, H.J.** ; **KIM, S.W.** ; **KANG, J.W.;** ; **HAN, S.J.** ; ; **JEONG, D.** ; **PARK, S.C.** Isolation and characterisation of pVa-21, a giant bacteriophage with anti-biofilm potential against Vibrio alginolyticus.. *Sci. Rep.*, 2019, vol. 9, 6284
- **ADAMS, M.H.** Bacteriophages.. Inter Science Publishers., 1959
- **BORN Y** ; **BOSSHARD L** ; **DUFFY B** ; **LOESSNER MJ** ; **FIESELER L.** Protection of Erwinia amylovora bacteriophage Y2 from UV-induced damage by natural compounds.. *Bacteriophage.*, 24 July 2015, vol. 5 (4), e1074330

- **JONES JB** ; **VALLAD GE** ; **IRIARTE FB** ; **OBRA-DOVIĆ A** ; **WERNSING MH** ; **JACKSON LE** ; **BALOGH B** ; **HONG JC** ; **MOMOL MT**. Considerations for using bacteriophages for plant disease control.. *Bacteriophage*., 01 October 2012, vol. 2 (4), 208-214
- **KAISER D** ; **BACHER S** ; **MÈNE-SAFFRANÉ L** ; **GRABENWEGER G.** Efficiency of natural substances to protect Beauveria bassiana conidia from UV radiation.. *Pest Manag Sci.*, February 2019, vol. 75 (2), 556-563
- **PARCEY M** ; **GAYDER S** ; **CASTLE AJ** ; **SVIRCEV AM**. Molecular Profile of Phage Infection: A Novel Approach for the Characterization of Erwinia Phages through qPCR. *Int J Mol Sci.*, 15 January 2020, vol. 21 (2), 553
- **GAYDER, S** ; **PARCEY, M** ; **NESBITT, D.** ; **CASTLE, A.J** ; **SVIRCEV, A.M.** Population Dynamics between Erwinia amylovora, Pantoea agglomerans and Bacteriophages: Exploiting Synergy and Competition to Improve Phage Cocktail Efficacy.. *Microorganisms*, 2020, vol. 8, 1449